# FASCICULE DE BREVET EUROPEEN

(11) **EP 1 222 159 B1**
(45) Date de publication et mention de la délivrance du brevet: **01.06.2005**
(21) Numéro de dépôt: 00971474.2
(22) Date de dépôt: 23.10.2000
(51) Int. Cl.: C07C 201/08, C07C 319/20, C07C 319/14, C07C 67/31, C07C 69/712, C07C 205/37, C07C 323/52, C07D 307/79, C07D 333/54

(54) **INTERMEDIAIRES DE FABRICATION D'UN DERIVE DE TYPE BENZOFURANE OU BENZOTHIOPHENE NITRE EN POSITION 5 ET LEURS UTILISATIONS**
ZWISCHENPRODUKTE FÜR DIE HERSTELLUNG VON 5- NITRO- BENZOFURAN ODER BENZOTHIOPHENDERIVATEN UND IHRE VERWENDUNGEN
INTERMEDIATES FOR MAKING A BENZOFURAN OR BENZOTHIOPHENE DERIVATIVE NITRATED IN POSITION 5 AND USES THEREOF

(30) Priorité: 21.10.1999 FR 9913250
(43) Date de publication de la demande: 17.07.2002
(73) Titulaire: RHODIA CHIMIE, 92408 Courbevoie Cédex (FR)
(72) Inventeur: SCHLAMA, Thierry, F-69570 Dardilly (FR); METTLING, Armand Résidence Charles X - Bât. B, F-68100 Mulhouse (FR); KARRER, Philippe, F-68720 Zillisheim (FR)
(74) Mandataire: Dutruc-Rosset, Marie-Claude
(86) Numéro de dépôt international: PCT/FR2000/002937
(87) Numéro de publication internationale: WO 2001/028974

(56) Documents cités:
- EP-A1- 0 129 034
- WO-A1-97/24122
- DE-A1- 3 031 738
- PATEL, VINOD F. ET AL: "Total Synthesis of Seco (+)- and ent-(-)-Oxaduocarmycin SA: Construction of the (Chloromethyl)indoline Alkylating Subunit by a Novel Intramolecular Aryl Radical Cyclization onto a Vinyl Chloride" J. ORG. CHEM. (1997), 62(25), 8868-8874, XP002142466
- BORDIN, FRANCO ET AL: "Nitrazione di alcuni benzofurani" GAZZ. CHIM. ITAL. (1969), 99(11), 1177-92, XP002142467
- CHEMICAL ABSTRACTS, vol. 116, no. 5, 3 février 1992 (1992-02-03) Columbus, Ohio, US; abstract no. 41094, KWIECIEN, HALINA ET AL: "Method for manufacturing 2-(o-formylphenoxy)hexanoic acid by hydrolysis of its methyl ester" XP002142471 & PL 152 990 B1 (POLITECHNIKA SZCZECINSKA, POL.) 28 Février 1991
- EDWARDS, COLIN R. ET AL: "A practical synthesis of 2,3-dihydro-2-benzofurancarboxylic acid. A general route to 2,3-dihydrobenzofurans" J. HETEROCYCL. CHEM. (1987), 24(2), 495-6, XP002142470
- SUZUKI, TSUNEO ET AL: "Benzofuran derivatives. I. On the effects of substituents in benzofuran syntheses" BULL. CHEM. SOC. JPN. (1983), 56(9), 2762-7, XP002142469
- JACOBS, WALTER A ET AL: "On nitro- and aminophenoxyacetic acids" JOURNAL OF THE AMERICAN CHEMICAL SOCIETY., vol. 39, no. 8, pages 2188-2224, XP000924885 AMERICAN CHEMICAL SOCIETY, WASHINGTON, DC., US ISSN: 0002-7863
- HULLAR, THEODORE L. ET AL: "Pyridoxal phosphate. II. Benzene analogs. 2-Formylphenoxyacetic acids as potential antimetabolites of pyridoxal phosphate" J. MED. CHEM. (1969), 12(3), 420-4, XP002142468
- K FRIES ET AL: "Unterschungen in der Reihe des Thionaphtens" JUSTUS LIEBIGS ANNALEN DER CHEMIE,XX,XX, vol. 527, no. 527, 1937, pages 83-114-110, XP002087887
- DATABASE WPI Section Ch, Week 198128 Derwent Publications Ltd., London, GB; Class C03, AN 1981-50551D XP002142473 & JP 56 059718 A (IHARA CHEM IND CO LTD), 23 mai 1981 (1981-05-23)
- CHEMICAL ABSTRACTS, vol. 99, no. 9, 29 août 1983 (1983-08-29) Columbus, Ohio, US; abstract no. 70551, FODOR, TAMAS ET AL: "2-Ethylbenzofuran" XP002142472 & HU 24 005 A (EGYT GYOGYSZERVEGYESZETI GYAR, HUNG.) 29 Novembre 1982

## Description

La présente invention a pour objet de nouveaux composés nitroaromatiques et leur procédé de préparation.

L'invention vise également l'utilisation de ces composés pour la préparation de dérivés hétérocycliques de type benzofurane ou benzothiophène. nitrés en position 5.

L'invention vise en particulier la préparation de 2-alkyl-5-nitrobenzofurane.

Des structures de type benzofurane ou benzothiophène se rencontrent dans de nombreuses molécules utilisées dans le domaine pharmaceutique. En particulier, on a décrit dans EP-A-0 471 609, un procédé de préparation de n-butyl-2-nitro-5-benzofurane qui consiste à faire réagir le bromure de 2-hydroxy-5-nitro-benzyltriphénylphosphonium avec le chlorure de pentanoyle en présence de pyridine : le bromure de 2-hydroxy-5-nitro-benzyltriphénylphosphonium étant obtenu à partir du bromure de 2-hydroxy-5-nitrobenzyle et de triphénylphosphine

Il a maintenant été trouvé une voie de synthèse totalement différente faisant intervenir des produits intermédiaires différents.

Par ailleurs, il est connu selon Jacobs et al (J.A.C.S. 39, 8, p. 2212) d'effectuer la nitration de l'acide o-aldéhydophénoxyacétique, à l'aide d'acide nitrique fumant à une température inférieure à 5°C.

La présente invention a pour objet de nouveaux composés nitroaromatiques répondant à la formule générale : 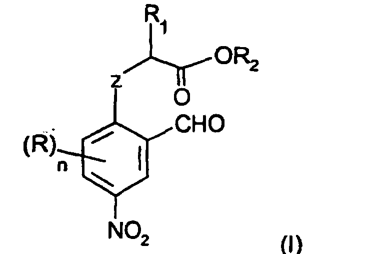 dans laquelle :
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle.
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

Dans la formule (I), le cycle benzénique peut porter un substituant.

L'invention n'exclut pas la présence sur le cycle benzénique de tout type de substituant dans la mesure où il ne réagisse pas dans les conditions de l'invention.

Comme exemples plus particuliers de groupes R, on peut mentionner entre autres :
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

Les composés préférés du procédé de l'invention répondent à la formule (I) dans laquelle R représente un atome d'hydrogène, un groupe méthyle ou éthyle, un groupe méthoxy ou éthoxy.

Lorsque n est supérieur à 1, deux groupes R et les 2 atomes successifs du cycle benzénique peuvent former entre eux un cycle saturé, insaturé ou aromatique ayant de 5 à 7 atomes de carbone, de préférence 6 atomes de carbone. Ainsi, deux groupes R forment avantageusement un cycle benzénique.

Le groupe R₁ est avantageusement un groupe alkyle ayant de 1 à 4 atomes de carbone.

L'invention n'exclut pas le fait que R₂ représente un autre groupe tel que cycloalkyle, phényle ou arylalkyle mais le groupe R₂ étant éliminé, il est intéressant d'un point de vue économique qu'il soit le plus simple possible par exemple, un groupe alkyle inférieur c'est-à-dire ayant de 1 à 4 atomes de carbone. R₂ peut également représenter un atome d'hydrogène ce qui correspond à la présence d'un groupe carboxylique.

Dans la formule (I), Z représente préférentiellement un atome d'oxygène.

Un autre objet de la présente invention est le procédé de préparation des composés nitroaromatiques de formule (I): 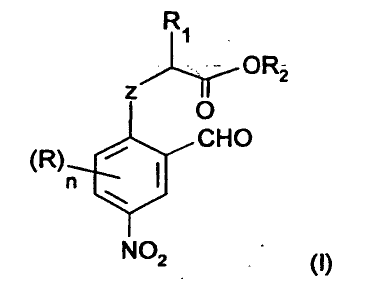 dans laquelle :
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle.
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone,
caractérisé par le fait qu'il consiste à effectuer la nitration sélective en position 4 à l'aide d'une source de NO₂⁺ et en présence d'acide sulfurique, d'un composé aromatique répondant à la formule (II) : 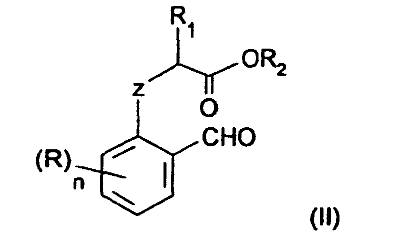 dans ladite formule (II), R, R₁, R₂, Z et n ont la signification donnée précédemment.

L'invention vise aussi bien la nitration d'un composé de formule (II) sous forme acide ou sous forme ester c'est-à-dire un composé de formule (II) dans laquelle R₂ est aussi bien un atome d'hydrogène qu'un groupe hydrocarboné.

Selon une variante préférée du procédé de l'invention, le composé de formule (II) est obtenu selon un procédé qui consiste à faire réagir :
- un composé de type 2-hydroxy- ou 2-thiobenzaldéhyde de formule (III) : 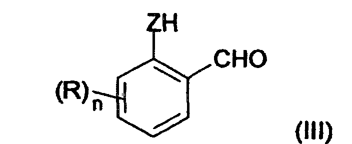 dans ladite formule (III), R, Z et n ont la signification donnée précédemment,
- et un un acide carboxylique ou dérivé de formule (IV) comprenant un groupe partant: 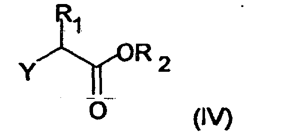 dans ladite formule (IV) :
   - Y représente un groupe partant, de préférence, un atome d'halogène ou un groupe ester suffonique de formule - OSO₂ - 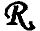 dans lequel 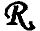 est un groupe hydrocarboné,
   - R₁ , R₂ ont la signification donnée précédemment.

Dans la formule du groupe ester sulfonique, 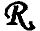 est un groupe hydrocarboné d'une nature quelconque. Toutefois, étant donné que Y est un groupe partant, il est intéressant d'un point de vue économique que 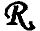 soit d'une nature simple, et représente plus particulièrement un groupe alkyle linéaire ou ramifié ayant de 1 à 4 atomes de carbone, de préférence, un groupe méthyle ou éthyle mais il peut également représenter par exemple un groupe phényle ou tolyle ou un groupe trifluorométhyle. Parmi les groupes Y, le groupe préféré est un groupe triflate ce qui correspond à un groupe 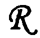 représentant un groupe trifluorométhyle.

Comme groupes partants préférés, on choisit de préférence, les atomes d'halogène, brome, chlore ou iode et de préférence un atome de brome ou de chlore.

L'invention vise également l'utilisation du composé de formule (I) pour la préparation d'un composé hétérocyclique répondant à la formule générale (V) : 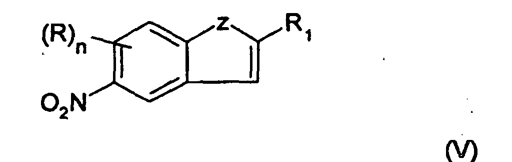 dans la formule (V), R, R₁, Z et n ont la signification donnée précédemment, par une saponification éventuellement quand R₂ est une fonction ester, suivie par une cyclisation.

Conformément au procédé de l'invention, on a préparé de nouveaux intermédiaires de fabrication répondant à la formule (I).

Ils ont été obtenus selon une réaction de nitration sélectivement en position 4 de composés répondant à la formule (II) dans laquelle R₁ est un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle.

Il a été trouvé qu'il n'était possible d'effectuer une réaction sélective de nitration en position para de l'atome O ou S que dans la mesure où l'on partait d'un substrat phénolique ou thiophénolique O- ou S- alkylé.

En effet, il a été trouvé que la nitration n'était pas sélective si l'on partait d'un substrat phénolique ayant des groupes OH ou SH libres.

Il a également été trouvé que la nitration n'était effectuée dans de bonnes conditions que dans la mesure où l'on associait la source de NO₂⁺ à l'acide sulfurique.

Le procédé de nitration sélectif selon l'invention s'applique à la préparation de nouveaux composés de formule (I) dans laquelle R₁ représente un groupe alkyle linéaire ou ramifié ayant de 1 à 12 atomes de carbone; un groupe phényle éventuellement substitué par un groupe alkyle ayant de 1 à 4 atomes de carbone ou un groupe halogénophényle

Ainsi, les composés de formule (I) peuvent être obtenus par nitration d'un composé de formule (II) O- ou S-alkylé, par réaction de ce dernier avec une source de NO₂⁺, de préférence, en présence ou non d'un solvant organique.

A cet effet, on fait réagir ledit composé avec une source de NO₂⁺.

Ainsi, on peut partir du dioxyde d'azote NO₂, de l'anhydride azoteux N₂O₃, du peroxyde d'azote N₂O₄, de l'oxyde d'azote NO associé à un agent oxydant tel que, par exemple, l'acide nitrique, le dioxyde d'azote ou l'oxygène. Dans le cas où le réactif est gazeux dans les conditions réactionnelles, on le fait buller dans le milieu.

On peut également faire appel à l'acide nitreux, à un sulfate de nitrosyle ou nitrose ou à un sel nitreux, de préférence un sel de métal alcalin, et encore plus préférentiellement, le sodium associé à un agent oxydant, de préférence l'acide nitrique.

Il est également possible de mettre en oeuvre des nitrites d'alkyle associés à un agent oxydant et plus particulièrement ceux répondant à la formule (VI) :

Rₐ- ONO (VI)

dans ladite formule (VI), Rₐ représente un groupe alkyle linaire ou ramifié ayant de 1 à 12 atomes de carbone, de préférence, de 1 à 4 atomes de carbone.

La quantité de source de NO₂⁺ est au moins égale à la quantité stoechiométrique du composé aromatique O- ou S-alkylé. Ainsi, le rapport entre le nombre de moles de source de NO₂⁺ et le nombre de moles de composé aromatique O- ou S-alkylé est compris avantageusement entre 1,0 et 1,2.

On fait appel préférentiellement à une solution d'acide nitrique concentrée ayant une concentration préférentielle comprise entre 70 et 99 %.

Comme mentionné précédemment, la source de NO₂⁺ est associée à l'acide sulfurique.

Une variante du procédé de l'invention consiste à faire appel à un mélange sulfonitrique (mélange d'acide nitrique et d'acide sulfurique comprenant de 50 à 98 % en poids d'acide nitrique).

La quantité d'acide nitrique exprimée par le rapport molaire composé aromatique O- ou S-alkylé/acide nitrique varie généralement entre 0,9 et 1,1, de préférence, entre 0,95 et 1,05.

La quantité d'acide sulfurique exprimée par le rapport molaire composé aromatique O- ou S-alkylé/acide sulfurique varie généralement entre 0,9 et 1,1, de préférence, entre 0,95 et 1,05.

La concentration en acide sulfurique est avantageusement comprise entre 50 % et 98 %.

A cet effet, on fait appel à l'acide nitrique ou à un précurseur de l'acide nitrique tel que par exemple le peroxyde d'azote.

La réaction de nitration peut être éventuellement conduite dans un solvant organique qui soit inerte dans les conditions réactionnelles.

Comme exemples plus particuliers de solvants organiques, on peut citer les hydrocarbures halogénés aliphatiques et plus particulièrement, les hydrocarbures perchlorés tels que notamment le tétrachlorométhane, l'hexachloroéthane ; les hydrocarbures partiellement chlorés tels que le dichlorométhane, ou le 1,2-dichloroéthane.

Le dichlorométhane est le solvant préféré.

En ce qui concerne la concentration du composé aromatique O- et S-alkylé, dans le milieu réactionnel, elle est de préférence comprise entre 0,2 et 3 mol/l et de préférence entre 0,3 et 1,5 mol/l.

Celui-ci est introduit généralement sous forme liquide.

La réaction est avantageusement effectuée à une température se situant entre entre -10°C et 20°C, de préférence entre -5°C et 10°C, et sous atmosphère de gaz inertes.

Le procédé de l'invention est généralement mis en oeuvre sous pression atmosphérique.

Selon une variante préférée du procédé de l'invention, on conduit l'étape de nitration sous atmosphère contrôlée de gaz inertes. On peut établir une atmosphère de gaz rares, de préférence l'argon mais il est plus économique de faire appel à l'azote.

Plusieurs modes de mises en oeuvre peuvent être envisagés.

Une première variante consiste à charger d'abord la solution d'acide sulfurique puis d'ajouter ensuite en parallèle le composé aromatique O- ou S-alkylé et l'acide nitrique.

Selon une autre variante, on introduit la solution d'acide sulfurique et l'acide nitrique puis l'on ajoute le composé aromatique O- ou S-alkylé, de préférence par portions ou de manière continue par coulée.

Une autre variante réside dans le fait d'introduire en parallèle, sur un pied de cuve, le composé aromatique O- ou S-alkylé d'un côté et l'acide sulfurique et l'acide nitrique de l'autre.

La réaction dure avantageusement entre 3 et 10 heures.

En fin de réaction, on obtient le produit désiré répondant à la formule (I).

On récupère le produit selon les techniques classiques utilisées dans le domaine technique.

On peut en particulier effectuer une hydrolyse à l'eau, de préférence, avec de la glace mise en oeuvre à raison par exemple de 100 à 150 % du poids du composé de formule (I).

On obtient un solide qui est séparé selon les techniques classiques de séparation solide/liquide, de préférence, par filtration.

On obtient ainsi le produit souhaité.

Conformément au procédé de l'invention, on part d'un composé de formule (II) qui peut être obtenu notamment selon une réaction de O- ou S-alkylation d'un composé de type 2-hydroxy- ou 2-thiobenzaldéhyde de formule (III) avec un un acide carboxylique ou dérivé de formule (IV).

Un premier mode de réalisation consiste à faire réagir un composé aromatique de formule (III) avec un acide carboxylique ou dérivé de formule (IV) : la réaction étant conduite en présence d'une base, de préférence dans un solvant organique.

Une autre variante du procédé de l'invention consiste à conduire la réaction de O- ou de S-alkylation, en milieu aqueux, en présence d'une base et d'un catalyseur de transfert de phase.

Parmi les composés de formule (III), l'aldéhyde salicylique est préféré.

Pour ce qui est l'acide carboxylique ou dérivé de formule (IV), on fait appel préférentiellement aux esters des acides α-halogénocarboxyliques et plus préférentiellement au 2-bromohexanoate de méthyle ou d'éthyle.

Le rapport molaire entre le composé de formule (III) et le composé de formule (IV) est avantageusement choisi entre 1 et 1,2.

Conformément au procédé de l'invention, on fait réagir le composé de type 2-hydroxy- ou 2-thiobenzaldéhyde de formule (III) sous forme salifiée et l'acide carboxylique ou dérivé de formule (IV), dans un solvant organique.

On peut faire appel à une forme salifiée d'un composé de type 2-hydroxy- ou 2-thiobenzaldéhyde préparée extemporanément mais il est également possible de le préparer in situ en faisant réagir le composé de type 2-hydroxy- ou 2-thiobenzaldéhyde et la base.

Intervient donc, dans le procédé de l'invention, une base qui peut être minérale ou organique.

Conviennent particulièrement bien à la mise en oeuvre du procédé de l'invention, les bases minérales telles que les sels de métaux alcalins ou alcalino-terreux, de préférence, un hydroxyde de métal alcalin ou alcalino-terreux qui peut être l'hydroxyde de sodium, de potassium ou de calcium ; un carbonate ou hydrogénocarbonate d'un métal alcalin, de préférence, le carbonate de sodium.

Il est également possible de faire appel à une base organique telle qu'un hydroxyde d'ammonium quaternaire ou à une amine.

Comme exemples d'hydroxyde d'ammonium quaternaire, on met en oeuvre préférentiellement, les hydroxydes de tétralkylammonium ou de trialkylbenzylammonium dont les groupes alkyle identiques ou différents représentent une chaîne alkyle linéaire ou ramifiée ayant de 1 à 12 atomes de carbone, de préférence de 1 à 6 atomes de carbone.

On choisit préférentiellement l'hydroxyde de tétraméthylammonium, l'hydroxyde de tétraéthylammonium ou l'hydroxyde de tétrabutylammonium.

Il est également possible selon l'invention d'avoir recours à des hydroxydes de trialkylbenzylammonium et notamment à l'hydroxyde de triméthylbenzylammonium.

Comme exemples d'amines, on peut mentionner entre autres les amines tertiaires.

Parmi les bases utilisables, il convient de citer les amines tertiaires et plus particulièrement celles répondant à la formule générale (VII)

N-(R₃)₃ (VII)

dans laquelle :
- les groupes R₃, identiques ou différents, représentent des restes hydrocarbonés ayant de 1 à 20 atomes de carbone, tels que des groupes alkyle, cycloalkyle, aryle ou hétérocyclique ;
- 2 groupes R₃ forment ensemble et avec l'atome d'azote un hétérocycle ayant 4 à 6 atomes.

Plus particulièrement :
- les symboles R₃ représentent un groupe alkyle ayant 1 à 10 atomes de carbone et de préférence 1 à 4 atomes de carbone ou un groupe cyclopentyle ou cyclohexyle ou un groupe pyridinyle ;
- 2 groupes R₃ forment ensemble et avec l'atome d'azote un cycle pipéridine ou pyrrolidine.

A titre d'exemples de telles amines, on peut citer la triéthylamine, la tri-*n*-propylamine, la tri-*n*-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-*n*-butylpipéridine, la 1,2-diméthylpipéridine, la N-méthylpyrrolidine, la 1,2-diméthylpyrrolidine.

Pour des considérations économiques, on choisit parmi toutes les bases, préférentiellement le carbonate de sodium ou de potassium.

Bien que la forme de mise en oeuvre de la base soit sous forme solide, on peut également faire appel à une base en solution La concentration de la solution basique de départ n'est pas critique. La solution d'hydroxyde de métal alcalin mise en oeuvre a une concentration généralement comprise entre 10 et 50 % en poids.

La quantité de base introduite dans le milieu réactionnel tient compte de la quantité nécessaire pour salifier la fonction hydroxyle ou thiol du composé de type 2-hydroxy- ou 2-thiobenzaldéhyde.

On peut effectuer la salification du groupe hydroxyle ou thiol du substrat de départ de formule (III) éventuellement dans une étape préalable. On peut donc salifier le composé de formule (III) soit en introduisant la base puis en la faisant réagir à une température avantageusement comprise entre 0°C et 100°C, de préférence, entre 25°C et 50°C, soit en introduisant la base en même temps que le composé de formule (IV).

Généralement, la quantité de base exprimée par rapport au composé de type 2-hydroxy- ou 2-thiobenzaldéhyde varie entre 90 et 120 % de la quantité stoechiométrique.

Conformément à l'invention, la réaction de O- ou S-alkylation est conduite avantageusement en phase liquide comprenant le composé de formule (III) et le composé de formule (IV), en présence d'une base.

L'un des réactifs de départ peut servir de solvant réactionnel mais il est également possible de faire appel à un solvant organique.

On choisit un solvant organique, moins activé que le substrat de départ et qui de préférence le solubilise.

Comme exemples de solvants convenant à la présente invention, on peut citer en particulier les hydrocarbures aromatiques, halogénés ou non, les éther-oxydes aliphatiques, cycloaliphatiques ou aromatiques.

A titre d'exemples d'hydrocarbures aliphatiques, on peut citer plus les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso.

En ce qui concerne les hydrocarbures halogénés aliphatiques ou aromatiques, on peut mentionner plus particulièrement, le dichlorométhane, le 1,2-dichloroéthane, le mono- ou dichlorobenzène.

On peut utiliser également à titre de solvants organiques, les éther-oxydes aliphatiques, cycloaliphatiques ou aromatiques et, plus particulièrement, l'oxyde de dipropyle, l'oxyde de diisopropyle, l'oxyde de dibutyle, le méthyltertiobutyléther, le diméthyléther de l'éthylèneglycol (ou glyme), le diméthyléther du diéthylèneglycol (ou diglyme) ; l'oxyde de phényle ; le dioxane, le tétrahydrofurane (THF).

Comme exemples de solvants organiques aprotiques, plus polaires qui peuvent également être mis en oeuvre dans le procédé de l'invention, on peut citer plus particulièrement les nitriles alphatiques ou aromatiques comme l'acétonitrile, le propionitrile, le benzonitrile ; les carboxamides linéaires ou cycliques comme le N,N-diméthylacétamide (DMAC), le diméthytformamide (DMF), le diéthylformamide ou la 1-méthyl-2-pyrrolidinone (NMP).

Les solvants préférés sont le DMAC ou le DMF.

On peut également utiliser un mélange de solvants organiques.

En ce qui concerne la concentration du composé de type 2-hydroxy- ou 2-thiobenzaldéhyde dans le milieu réactionnel, elle est de préférence comprise entre 2 % et 50 % en poids.

Une variante du procédé de l'invention consiste à ajouter des ions iodure afin d'accélérer la réaction. Ainsi, on peut faire appel notamment à des iodures de métaux alcalins, de préférence l'iodure de potassium ou à des iodures de tétralkylammonium, de préférence l'iodure de tétrabutylammonium.

La quantité de iodure mise en jeu, exprimée par le rapport entre le nombre de moles de sel iodé et le nombre de moles de composé de formule (III) peut varier entre 0,05 et 0,2.

La température de la réaction du composé aromatique de formule (III) avec un acide carboxylique ou dérivé de formule (IV) est avantageusement comprise entre 0°C et 100°C, de préférence, entre 25°C et 50°C.

La réaction a lieu généralement sous pression atmosphérique.

Selon une variante préférée du procédé de l'invention, on conduit le procédé de l'invention, sous atmosphère contrôlée de gaz inertes. On peut établir une atmosphère de gaz rares, de préférence l'argon mais il est plus. économique de faire appel à l'azote.

D'un point de vue pratique, le procédé est simple à mettre en oeuvre.

Un mode de réalisation de l'invention consiste à charger tous les réactifs, la base, le solvant organique éventuellement les ions iodure.

On porte le milieu à la température réactionnelle choisie.

Comme mentionné précédemment, on peut effectuer la salification dans une étape préalable et introduire le composé de formule (III) la base et le solvant organique, porter le milieu à la température choisie puis ajouter le composé de formule (IV) et éventuellement des ions iodure puis on chauffe.

On obtient le produit désiré qui répond à la formule (II).

On récupère le produit obtenu d'une manière classique.

On peut par exemple éliminer les sels formés au cours de la réaction par addition d'eau et extraire ledit produit en phase organique, dans un solvant adéquat par exemple l'éther isopropylique.

Le solvant organique peut être éliminé d'une manière classique par évaporation.

Selon une variante du procédé de l'invention, on fait réagir le composé de type 2-hydroxy- ou 2-thiobenzaldéhyde de formule (III) avec un acide carboxylique ou dérivé de formule (IV), en milieu aqueux, en présence d'une base et d'un catalyseur de transfert de phase.

Par l'expression "catalyseur de transfert de phase", on entend un catalyseur capable de faire passer l'anion de la phase aqueuse à la phase organique.

Dans le procédé de l'invention, on peut faire appel aux catalyseurs de transfert de phase connus, notamment, ceux décrits dans l'ouvrage de Jerry MARCH - Advanced Organic Chemistry, 3ème édition, John Wiley & Sons, 1985 p. 320 et suivantes.

Une première catégorie de catalyseurs de transfert de phase convenant à l'invention sont ceux de type tris(éther-amines) qui sont décrits dans la littérature et notamment dans FR-A 2 455 570.

Ils répondent à la formule suivante :

N⁅A-O-(-B-O-)ₙ-R_{b}]₃ (VIII)

dans ladite formule, R_{b} représente un groupe alkyle comprenant de 1 à 24 atomes de carbone, un groupe cyclohexyle, un groupe phényle, un groupe alkylphényle dont la partie alkyle comprend de 1 à 12 atomes de carbone, A et B, identiques ou différents, représentent un groupement alcanediyle linéaire contenant 2 ou 3 atomes de carbone, lesdits atomes pouvant être substitués par un groupe méthyle ou éthyle.

Comme exemples spécifiques de catalyseurs répondant à la formule (VIII), on peut mentionner tout particulièrement la tris(3,3-dioxaheptyl)amine (TDA-1).

Les catalyseurs préférentiellement mis en oeuvre dans le procédé de l'invention sont les sels d'onium et, plus particulièrement les sels d'ammonium quaternaire et/ou de phosphonium.

Les sels d'omnium susceptibles d'être utilisés dans le procédé de l'invention sont ceux dont les ions onium dérivent- notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène ou du carbone seront tricoordinés tandis que les ions onium dérivant de l'iode seront dicoordinés.

Les restes hydrocarbonés coordinés à ces différents éléments sont des groupes alkyle, alcényle, aryle, cycloalkyle, aralkyle éventuellement substitués, deux restes hydrocarbonés coordinés pouvant former ensemble un groupe unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581 - 587 (1968), les termes "dure" et "intermédiaire" ayant respectivement la signification des termes de "hard" et "borderline" utilisés dans cette référence.

Parmi les ions onium pouvant être utilisés dans le présent procédé de l'invention, conviennent particulièrement ceux répondant à l'une des formules générales suivantes : 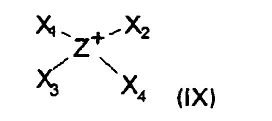 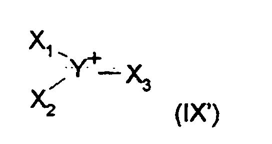 dans lesdites formules :
- Z représente N, P ou As ;
- Y représente S, O, Se ou C ;
- X₁, X₂, X₃ et X₄, identiques ou différents représentent :
   . un groupe alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
   . un groupe alcényle, linéaire ou ramifié, ayant 2 à 12 atomes de carbone ;
   . un groupe aryle ayant 6 à 10 atomes de carbone, éventuellement substitué par un ou plusieurs groupements ou atomes alkyle ayant 1 à 4 atomes de carbone, alkoxy, alkoxycarbonyle, le groupe alkoxy ayant 1 à 4 atomes de carbone, ou halogène,
- deux desdits groupes X₁ à X₄ pouvant former ensemble un groupe alkylène, alcénylène ou alcadiènylène, linéaire ou ramifié ayant de 3 à 6 atomes de carbone.

Parmi les bases "dures" ou "intermédiaires" pouvant constituer l'anion desdits sels d'onium, on peut citer les ions : F⁻, ClO₄⁻, PF₆⁻, BF₄⁻, SnCl₆⁻, SbCl₆⁻, B(Ph)₄⁻, PO₄³⁻, HPO₄²⁻, H₂PO₄⁻, CH₃SO₃⁻, Ph-SO₃⁻, HSO₄⁻, NO₃⁻, SO₄²⁻, Cl⁻, Br⁻, I⁻, OH⁻, Ph représentant un groupe phényle, ainsi que tous les autres anions répondant à la définition de base "dure" ou "intermédiaire" de PEARSON.

Pour des raisons de commodité de mise en oeuvre, lesdits anions pourront être choisis parmi PO₄³⁻, HPO₄²⁻, H₃PO₄⁻, CH₃SO₃⁻, Ph-SO₃⁻, NO₃⁻, SO₄²⁻, PF₆⁻, Cl⁻, Br⁻, I⁻, Ph ayant la signification précédente. On choisit avantageusement les anions Br⁻ et Cl⁻.

A titre d'exemples d'ions onium répondant à la formule (IX), on peut citer les cations :
- tributylrnéthylammonium,
- tétraéthylammonium,
- tétrabutylammonium,
- dodécyltriméthylammonium,
- méthyltrioctylammonium,
- heptyltributylammonium,
- tétrahexylammonium,
- tétraheptylammonium,
- tétraoctylammonium,
- benzyltriméthylammonium,
- benzyldiméthylpropylammonium,
- benzyldiméthyloctylammonium,
- benzyltributylammonium,
- benzyltriéthylammonium,
- phényltriméthylammonium,
- benzyldiméthyltétradécylammonium,
- benzyldiméthylhexadécylammonium,
- tétrabutylphosphonium,
- triméthylpentylphosphonium,
- triméthylphénylphosphonium,
- diéthyldiméthylphosphonium,
- dicyclohexyldiméthylphosphonium,
- diméthyldiphénylphosphonium,
- cyclohexyltriméthylphosphonium,
- méthyftribenzylphosphonium,
- méthyitri(méthyl-4 phényl) phosphonium,
- éthyltri(n-propyl)phosphonium,
- triéthylpentylphosphonium,
- hexadécyltributylphosphonium,
- éthyltriphénylphosphonium,
- n-butyltri(n-propyl)phosphonium,
- tétraphénylphosphonium,
- triphényl(méthyl-4 phényl)phosphonium,
- tétrakis(hydroxyméthyl)phosphonium,
- tétraphénylarsonium.

A titre d'exemples d'ions onium répondant à la formule (IX'), on peut citer les cations :
- triéthylsulfonium,
- triphénylsulfonium,

Parmi les ions onium qui peuvent être utilisés dans le cadre du présent procédé, on préférera le plus souvent les ions ammonium quaternaire, les ions phosphonium quaternaire.

Conviennent tout particulièrement bien, les ions ammonium dont les quatre groupes sont des groupes alkyle ayant de 1 à 5 atomes de carbone ou un groupe benzyle.

En ce qui concerne le choix de l'anion, on préférera Br⁻, Cl⁻ ou OH⁻.

Les catalyseurs convenant tout à fait bien à la présente invention sont le chlorure ou le bromure de tributylbenzylammonium ou phosphonium, le chlorure ou le bromure de tétraméthylammonium ou phosphonium, le chlorure ou le bromure de tétraéthylammonium ou phosphonium, le chlorure ou le bromure de tétrabutylammonium ou phosphonium.

Le chlorure ou bromure de benzyttributlylammonium est particulièrement préféré, le dérivé chloré étant plus particulièrement préféré.

Le sel d'onium peut être introduit au cours du procédé de l'invention, à l'état solide ou sous forme d'une solution dans l'un de ses solvants, le plus souvent l'eau.

Le procédé selon l'invention est avantageusement conduit en l'absence de solvant.

Le procédé conforme à l'invention est effectué en présence d'une base soluble dans l'eau.

On utilise avantageusement le carbonate de potassium ou de sodium, ou l'ammoniaque.

Selon un mode de réalisation préférentiel, on utilise du carbonate de potassium.

La quantité de base mise en oeuvre exprimée par le rapport entre le nombre de moles du composé de formule (III) et le nombre de moles de base est choisie de préférence entre 1 et 5 et plus préférentiellement aux environs de 1,5.

Conformément au procédé de l'invention, la réaction de O- ou S-alkylation du composé de formule (III) est effectuée en présence d'un catalyseur de transfert de phase, les différents réactifs étant mis en oeuvre généralement dans les proportions définies ci-après.

Le rapport molaire entre le nombre de moles de composé de formule (III) et le nombre de moles de composé de formule (IV) varie de préférence entre 1 et 1,2.

En ce qui concerne la quantité de catalyseur utilisée, celle-ci varie avantageusement de telle sorte que le rapport molaire entre ledit catalyseur et le composé de formule (III) varie entre 0,01 et 0,50, de préférence, entre 0,05 et 0,2. La borne supérieure n'est pas critique et peut être largement dépassée sans inconvénient car le catalyseur peut être éventuellement recyclé en fin de réaction.

Comme mentionné précédemment, la réaction est conduite, en milieu aqueux, avantageusement en l'absence de tout solvant organique.

Selon un mode préférentiel de réalisation de l'invention, on choisit une concentration en composé de formule (III) aussi élevée que possible.

La quantité d'eau présente dans le milieu réactionnel représente généralement de 30 à 100 % du poids total des réactifs engagés.

La réaction est avantageusement effectuée selon le principe "one pot", l'ordre d'introduction des réactifs et corps réagissants n'étant pas critique.

La température à laquelle est mis en oeuvre le procédé de l'invention se situe généralement entre la température ambiante et 80°C. D'une manière préférentielle, la température est choisie entre 50°C et 65°C.

La pression réactionnelle n'est pas critique et est généralement la pression atmosphérique.

Pour atteindre les températures indiquées précédemment, on travaille de préférence sous pression autogène.

La durée de la réaction dépend de la température réactionnelle et du taux de transformation désiré. Lorsque la température est choisie dans la zone préférentielle, la durée de la réaction peut varier dans de larges limites, par exemple, de 6 à 10 heures.

En fin de réaction, le composé aromatique O- ou S-alkylé de formule (II) est contenu dans ou constitue la phase organique qui peut être séparée de la phase aqueuse, notamment par décantation.

On peut isoler le composé obtenu de la phase organique, selon les techniques classiquement utilisées telles que, par exemple, la distillation ou l'extraction à l'aide d'un solvant adéquat.

Conformément à l'invention, le composé de formule (I) est un produit intermédiaire pour la fabrication du composé de formule (V).

Dans une étape suivante, on effectue si nécessaire la saponification de la fonction ester en fonction carboxylique puis l'on effectue la cyclisation du produit obtenu. Selon une autre variante, il est possible d'effectuer de la même manière, la saponification si nécessaire du composé de formule (II), avant l'opération de nitration.

A cet effet, on fait donc réagir le composé de formule (I) avec une base en milieu hydro-organique.

Comme base, on fait appel préférentiellement à la soude ou à la potasse qui sont mises en oeuvre sous la forme de paillettes ou de solutions concentrées, par exemple à 40 % pour la soude.

La quantité de base mise en oeuvre exprimée par le rapport entre le nombre de moles du composé de formule (I) et le nombre de moles de base est choisie de préférence entre 1 et 5 et plus préférentiellement entre 1 et 2.

La base est solubilisée en milieu aqueux ou hydre-organique.

On fait appel de préférence à un solvant organique polaire.

Comme exemples de solvants organiques convenant à la présente invention, on peut citer plus particulièrement les alcools aliphatiques tels que l'éthanol, le propanol, le butanol, le pentanol, l'éthylène glycol ; les alcools cycloaliphatiques, notamment le cyclohexanol et les alcools arylaliphatiques, plus particulièrement l'alcool benzylique. On peut également envisager les éthers monométhylique, monoéthylique, monopropylique, monobutylique, de l'éthylène glycol vendus sous la dénomination commerciale de Cellosolves®.

La concentration du composé de formule (I) dans le milieu réactionnel (eau + solvants organiques) varie avantageusement entre 5 et 50 %, de préférence, entre 5 et 20 % en poids.

Le rapport volumique entre le solvant organique et l'eau peut varier, par exemple, entre 0,1 et 0,9, et il se situe de préférence, vers 0,1 et 0,2.

Le choix du solvant organique et du rapport eau/solvant organique est déterminé de telle sorte que la solution obtenue soit homogène.

On effectue la réaction de saponification à une température comprise entre la température ambiante et la température de reflux du mélange réactionnel, préférentiellement à une température voisine de 50°C.

Par température ambiante, on entend généralement une température comprise entre 15°C et 25°C.

Selon un mode de réalisation pratique de l'invention, on introduit le composé de formule (I) dans le milieu aqueux ou hydro-organique puis l'on additionne la base puis l'on amène le milieu réactionnel à la température choisie.

En fin de réaction, si nécessaire, on neutralise l'excès de base par une solution acide, de préférence, une solution d'acide minéral ou d'un sel minéral, tel que par exemple, l'acide chlorhydrique ou le chlorure d'ammonium.

Le produit obtenu précipite puis on le sépare selon les techniques classiques de séparation solide/liquide, de préférence, par filtration.

On peut effectuer la cyclisation du produit obtenu selon l'enseignement de l'état de la technique par exemple dans l'anhydride acétique et en présence d'acétate de sodium (Brady, W. T.; Gu, Y.-Q. J. Heterocycl.Chem. 1988, 25, 969-971 ).

Quant à la température de la réaction de cyclisation, elle est avantageusement choisie entre la température ambiante et la température de reflux du solvant réactionnel.

On obtient un dérivé de type benzofurane ou benzothiophène nitré en position 4 et répondant à la formule (V). 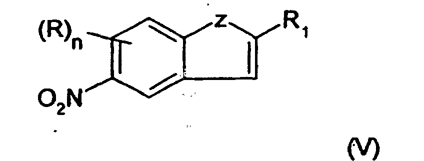 dans la formule (V), R, R₁, Z et n ont la signification donnée précédemment, par éventuelle saponification du composé de formule (I) quand R₂ est une fonction ester puis cyclisation.

Un autre mode de réalisation de la cyclisation du composé de formule (I) consiste à effectuer la cyclisation dans un milieu comprenant un anhydride d'acide carboxylique et en présence d'une base qui est choisie parmi les carbonates et/ou hydrogénocarbonates métalliques ou d'ammonium.

Comme bases susceptibles d'être utilisées, on peut mentionner tout particulièrement les carbonates et les hydrogénocarbonates de métaux alcalins ou alcalino-terreux. On peut faire appel au carbonate de césium mais l'on préfère le carbonate de sodium ou le carbonate de potassium.

Conformément au procédé de l'invention, on effectue la cyclisation du composé aromatique répondant de préférence, à la formule (I) dans un anhydride d'acide carboxylique.

Ce dernier répond plus particulièrement à la formule suivante : 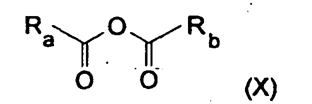 dans ladite formule (X) :
- Rₐ et R_{b}, identiques ou différents, représentent un groupe hydrocarboné monovalent, substitué ou non qui peut être un groupe aliphatique acyclique, saturé ou insaturé, linéaire ou ramifié ; un groupe carbocyclique saturé, insaturé ou aromatique, monocyclique,
- Rₐ et R_{b} pouvant former ensemble un groupe divalent aliphatique saturé
ou insaturé, linéaire ou ramifié, ayant au moins 2 atomes de carbone.

Les groupes Rₐ et R_{b} sont choisis préférentiellement de telle sorte que l'anhydride soit liquide dans les conditions réactionnelles.

L'anhydride mis en oeuvre peut être cyclique ou non.

Plus précisément, on fait appel à un anhydride cyclique ayant de 5 à 10 atomes dans le cycle ne comprenant pas ou comprenant une double liaison, l'un des atomes pouvant être remplacé par un atome d'oxygène.

Les anhydrides cycliques mis en oeuvre préférentiellement sont saturés ou comprennent une double liaison et possèdent 5 ou 6 atomes dans le cycle.

Le cycle peut comprendre un ou plusieurs substituants. Comme exemples plus particuliers de substituants, on peut citer, notamment les groupes alkyle, linéaires ou ramifiés, ayant de 1 à 12 atomes de carbone, de préférence de 1 à 4 atomes de carbone, tel que méthyle, éthyle, propyle, isopropyle, butyle, isobutyle, sec-butyle, tert-butyle ou des atomes d'halogène ou un groupe trihalogénométhyle.

Pour ce qui de la mise en oeuvre d'un anhydride non cyclique répondant à la formule (X), les groupes Rₐ et R_{b}, identiques ou différents représentent plus particulièrement :
- un groupe aliphatique acyclique linéaire ou ramifié ayant de préférence de 1 à 24, de préférence, de 1 à 12 atomes de carbone, saturé ou comprenant une à plusieurs insaturations sur la chaîne, généralement, 1 à 3 insaturations qui peuvent être des doubles liaisons simples : la chaîne hydrocarbonée pouvant être interrompue par l'un des groupes suivants : -O-; -CO- ; et/ou porteuse d'un ou plusieurs substituants notamment : -X ; -CX₃.
- un groupe carbocyclique, saturé, insaturé ou aromatique, ayant de 3 à 8 atomes de carbone, de préférence, 6 atomes de carbone, éventuellement porteur d'un ou plusieurs atomes d'halogène, de préférence, chlore ou brome.

Parmi les groupes précisés, Rₐ et R_{b} représentent préférentiellement :
- un groupe alkyle, linéaire ou ramifié comprenant de 1 à 12 atomes de carbone, éventuellement porteur d'un ou plusieurs atomes d'halogène,
- un groupe cyclohexyle ou phényle, éventuellement porteur d'un ou plusieurs atomes d'halogène, un groupe trihalogénométhyle.

A titre d'exemples d'anhydrides, on peut citer :
- l'anhydride acétique,
- l'anhydride propanoïque,
- l'anhydride isobutyrique,
- l'anhydride trichloroacétique,
- l'anhydride trifluoroacétique,
- l'anhydride benzoïque.
- l'anhydride de monochloroacétyle,
- l'anhydride de dichloroacétyle,
- l'anhydride pivalique.

Parmi les anhydrides précités, l'anhydride acétique est préféré.

L'invention n'exclut pas l'anhydride carboxylique soit engendré dans le milieu, à partir d'un acide carboxylique.

Une variante préférée du procédé de l'invention à un solvant organique.

Plusieurs impératifs président au choix du solvant organique.

Une première caractéristique du solvant organique est qu'il soit stable dans le milieu réactionnel.

Une deuxième caractéristique est que le solvant présente un point d'ébullition élevé, de préférence, supérieur ou égal à 50°C.

Comme exemples de solvants convenant à la présente invention, on peut citer en particulier les hydrocarbures aromatiques, halogénés ou non, les éther-oxydes aliphatiques, cycloaliphàtiques ou aromatiques. Des exemples de tels solvants sont donnés précédemment.

Comme exemples de solvants organiques aprotiques, plus polaires qui peuvent également être mis en oeuvre dans le procédé de l'invention, on peut citer plus particulièrement les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP) ; le diméthylsulfoxyde (DMSO) ; l'hexaméthylphosphotriamide (HMPT) ; la tétraméthylurée ; les composés nitrés tels que le nitrométhane, le nitroéthane, le 1-nitropropane, le 2-nitropropane ou leurs mélanges, le nitrobenzène; les nitriles alphatiques ou aromatiques comme l'acétonitrile, le propionitrile, le butanenitrile, l'isobutanenitrile, le benzonitrile, le cyanure de benzyle ; la tétraméthylène sulfone (sulfolane).

On peut également utiliser un mélange de solvants.

Selon le procédé de l'invention, la cyclisation du substrat de départ est conduite en présence d'une base et d'un anhydride d'acide carboxylique.

Plus précisément, la quantité de base exprimée par le rapport entre le nombre de moles de base et le nombre de moles de substrat de départ répondant préférentiellement à la formule (I) varie entre 0,05 et 1,0 et se situe de préférence, entre 0,1 et 0,2.

La quantité d'anhydride d'acide carboxylique mise en oeuvre est telle que le rapport molaire anhydride d'acide carboxylique/composé de formule (I) varie de préférence, entre 2 et 10.

Dans la variante préférée du procédé de l'invention qui consiste à mettre en oeuvre un solvant organique, la quantité d'anhydride d'acide carboxylique mise en oeuvre est telle que le rapport molaire anhydride d'acide carboxylique/composé de formule (I) varie de préférence, entre 1 et 3 et encore plus préférentiellement entre 1 et 2.

Pour ce qui est de la quantité de solvant organique à mettre en oeuvre, elle est déterminée en fonction de la nature du solvant organique choisi.

Elle est déterminée de telle sorte que la concentration du substrat dans le solvant organique soit de préférence comprise entre 1 et 10 mol/litre et plus préférentiellement entre 2 et 3 mol/litre.

La réaction de cyclisation du substrat de départ a lieu à une température qui est avantageusement située entre 50°C et 160°C, de préférence, entre 100°C et 140°C.

La réaction de cyclisation est généralement mise en oeuvre sous pression atmosphérique mais de préférence, sous atmosphère contrôlée de gaz inertes. On peut établir une atmosphère de gaz rares, de préférence l'argon mais il est plus économique de faire appel à l'azote.

D'un point de vue pratique, la réaction est simple à mettre en oeuvre.

L'ordre de mis en oeuvre des réactfs n'est pas critique. Une variante préférée consiste à charger le solvant organique si présent, le substrat, l'anhydride carboxylique et ensuite la base et l'on chauffe à la température souhaitée.

En fin de réaction, on obtient le produit cyclisé qui répond à la formule (V) et qui peut être récupéré d'une manière classique.

L'invention concerne plus particulièrement la préparation du 2-n-butyl-5-nitrobenzofurane.

Les exemples qui suivent illustrent l'invention sans pour autant la limiter.

### Exemples

### Exemple 1

### Préparation de l'acide 2-(2-formyl-4-nitro-phénoxy)-hexanoïque

L'acide 2-(2-formyl-4-nitro-phénoxy)-hexanoïque peut être préparé de la manière suivante :

Dans un réacteur tétracol de 250 ml, muni d'une agitation pale Téflon demi lune, d'un thermomètre, d'une ampoule d'addition de 50 ml, d'un réfrigérant à serpentin et d'une arrivée d'azote, on charge successivement 29,5 g de 2-(2-formyl-4-nitro-phénoxy)-hexanoate de méthyle, 148 ml d'eau.

On ajoute en 20 minutes, 8,4 g d'une solution aqueuse à 50 % d'hydroxyde de sodium.

Après 15 minutes sous agitation à une température voisine de 25°C, le milieu réactionnel est chauffé pendant 2 heures aux environs de 50°C.

Le milieu limpide obtenu de couleur rouge est partiellement évaporé (50 ml) sous pression réduite (20 mm Hg) pour éliminer le méthanol formé puis redilué par 50 ml d'eau.

Le pH du milieu réactionnel est amené au voisinage de 1,8 par addition lente de 10,8 g d'acide chlorhydrique concentré en maintenant la température au voisinage de 45°C sous agitation.

Après une heure sous agitation, la température du milieu est portée au voisinage de 55°C pendant 20 minutes puis le milieu est laissé à température ambiante pendant 12 heures.

Le produit solide est séparé par filtration sur verre fritté N°3 et lavé par 2 fois 50 ml d'eau puis séché à l'étuve pendant 12 heures à une température voisine de 55°C.

On obtient ainsi 26,8 g d'acide 2-(2-formyl-4-nitro-phénoxy)-hexanoïque sous forme d'un solide jaune clair fondant à 110-111°C et titrant 97,5 % par dosage potentiométrique.

Son spectre RMN est le suivant:
RMN ¹H (DMSO-d6) : δ 0,91 (t, 3H, CH₃) ; 1,38 (m, 2H, CH₂-CH₃) ; 1,51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1 H, CHO) ; 13,45 (pic large, 1H, COOH).

### Exemple 2

### Préparation de l'acide 2-(2-formyl-phénoxy)-hexanoïque.

L'acide 2-(2-formyl-phénoxy)-hexanoïque peut être préparé de la manière suivante :

Dans un réacteur tétracol de 250 ml, muni d'une agitation pale Téflon demi lune, d'un thermomètre, d'une ampoule d'addition de 50 ml, d'un réfrigérant à serpentin et d'une arrivée d'azote, on charge successivement 29,5 g de 2-(2-formyl-phénoxy)-hexanoate de méthyle, 148 ml d'eau.

On ajoute en 20 minutes, 10.4 g d'une solution aqueuse à 50 % d'hydroxyde de sodium.

Après 15 minutes sous agitation à une température voisine de 25°C, le milieu réactionnel est chauffé pendant 2 heures aux environs de 50°C.

Le milieu limpide obtenu est partiellement évaporé (50 ml) sous pression réduite (20 mm Hg) pour éliminer le méthanol formé puis redilué par 50 ml d'eau.

Le pH du milieu réactionnel est amené au voisinage de 1,8 par addition lente de 10,8 g d'acide chlorhydrique concentré en maintenant la température au voisinage de 45°C sous agitation.

Après une heure sous agitation, la température du milieu est portée au voisinage de 55°C pendant 20 minutes puis le milieu est laissé à température ambiante pendant 12 heures.

Le produit solide est séparé par filtration sur verre fritté N°3 et lavé par 2 fois 50 ml d'eau puis séché à l'étuve pendant 12 heures à une température voisine de 55°C.

On obtient ainsi 27.4 g d'acide 2-(2-formyl-phénoxy)-hexanoïque sous forme d'un solide jaune clair et titrant 98 % par dosage potentiométrique.

### Exemple 3

### Préparation du 2-(2-formyl-4-nitro-phénoxy)-hexanoate de méthyle.

Le 2-(2-formyl-4-nitro-phénoxy)-hexanoate de méthyle peut être préparé de la manière suivante :

Dans un ballon tétracol de 250 ml, muni d'une agitation pale Téflon demi lune, d'un thermomètre, d'une ampoule d'addition de 50 ml, d'un réfrigérant à serpentin et d'une arrivée d'azote, on charge 123 g d'acide sulfurique concentré à 96 %.

Le milieu réactionnel est refroidi à une température voisine de 5 °C puis on ajoute à cette même température 30 g (0,12 mole) de 2-(2-formyl-phénoxy)-hexanoate de méthyle.

Après 15 minutes sous agitation, on additionne en 2 heures, 15,9 g (0,126 mol) de sulfonitrique (50/50) en maintenant la température du milieu réactionnel au voisinage de 5°C puis on ajoute en 30 minutes 76,9 g de glace conduisant à un titre de 60% en H₂SO₄.

Le mélange réactionnel est filtré sur verre fritté N° 3 après 10 minutes d'agitation.

Le produit brut obtenu est dissous dans 100 ml de dichlorométhane, et lavé avec 2 fois 50 ml d'eau.

La phase organique décantée est concentrée à l'évaporateur rotatif de 20 à 70° C sous 20 mm de mercure (durée : 2 heures).

On obtient ainsi 32,7 g de produit solide jaune beige soit un rendement en 2-(2-formyl-4-nitro-phénoxy)-hexanoate de méthyle de 92,4 % titrant 96,7 % par chromatographie en phase gazeuse.

Son spectre RMN est le suivant :
RMN ¹H (DMSO-d6) : δ 0,91 (t, 3H, CH₃) ; 1,38 (m, 2H, CH₂-CH₃) ; 1,51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1H, CHO) ; 13,45 (pic large, 1H, COOH).

### Exemple 4

### Préparation de l'acide 2-(2-formyl-4-nitro-phénoxy)-hexanoïque.

L'acide 2-(2-formyl-4-nitro-phénoxy)-hexanoïque peut être préparé de la manière suivante :

Dans un ballon tétracol de 250 ml, muni d'une agitation pale Téflon demi lune, d'un thermomètre, d'une ampoule d'addition de 50 ml, d'un réfrigérant à serpentin et d'une arrivée d'azote, on charge 123 g d'acide sulfurique concentré à 96 %.

Le milieu réactionnel est refroidi à une température voisine de 5 °C puis on ajoute à cette même température 28.4 g (0,12 mole) d'acide 2-(2-formyl-phénoxy)-hexanoïque.

Après 15 minutes sous agitation, on additionne en 2 heures, 15,9 g (0,126 mol) de sulfonitrique (50/50) en maintenant la température du milieu réactionnel au voisinage de 5°C puis on ajoute en 30 minutes 76,9 g de glace conduisant à un titre de 60% en H₂SO₄.

Le mélange réactionnel est filtré sur verre fritté N° 3.

Le solide obtenu est dissous dans 100 ml de dichlorométhane, et lavé par 2 fois 50 ml d'eau.

La phase organique décantée est concentrée à l'évaporateur rotatif de 20 à 70° C sous 20 mm de mercure (durée : 2 heures).

On obtient ainsi 32,1 g de produit solide jaune beige soit un rendement en acide 2-(2-formyl-4-nitro-phénoxy)-hexanoïque de 95 % titrant 97,0 % par chromatographie en phase liquide.

Son-spectre RMN est le suivant :
RMN ¹H (DMSO-d6) : δ 0,91 (t, 3H, CH₃) ; 1,38 (m, 2H, CH₂-CH₃) ; 1,51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1H, CHO) ; 13,45 (pic large, 1H, COOH).

### Exemple 5

### Préparation du 2-(2-formyl-phénoxy)-hexanoate de méthyle.

Le 2-(2-formyl-phénoxy)-hexanoate de méthyle peut-être préparé de la manière suivante :

Dans un réacteur de 1 litre tétracol muni d'une agitation pale demi lune, d'un thermomètre, d'un réfrigérant à serpentin et d'une ampoule d'addition de 500 ml , on charge successivement sous atmosphère contrôlée 87,1 g (0,714 mol) d'aldéhyde salicylique, 158,2 g (0,756 mol) de 2-bromo-hexanoate de méthyle, 103,5 g (0,75 mol) de carbonate de potassium et 5,9 g (0,0355 mol) d'iodure de potassium.

On ajoute 400 g de diméthylformamide et l'ensemble est chauffé sous agitation à une température voisine de 80° C pendant 4 h.

Après retour à une température voisine de 25°C, le mélange réactionnel est filtré sur verre fritté n° 3 et lavé par 50 g de diméthylformamide.

Le filtrat est concentré par évaporation sous pression réduite (25-40 mbars) puis dilué par 100 ml d'eau et extrait, successivement, par 1 fois 100 ml de dichlorométhane et 50 ml de dichlorométhane.

Les phases organiques réunies sont lavées avec 50 ml d'eau et concentrées à sec par évaporation sous pression réduite.

On obtient ainsi 176,1 g de liquide jaune limpide correspondant à un rendement de 98,6 % en 2-[2-(formyl-phénoxy)]-hexanoate de méthyle titrant 99,6 % de pureté par chromatographie en phase gazeuse.

### Exemple 6

### Préparation du 2-(2-formyl-phénoxy)-hexanoate de méthyle.

Le 2-(2-formyl-phénoxy)-hexanoate de méthyle peut-être préparé de la manière suivante :

Dans un réacteur de 2 litres tétracol muni d'une agitation pale demi lune, d'un thermomètre, d'un réfrigérant à serpentin et d'une ampoule d'addition de 1000 ml , on charge successivement sous atmosphère contrôlée 130,6 g (1,071 mol) d'aldéhyde salicylique, 237,3 g (1,134 mol) de 2-bromo-hexanoate de méthyle, 155,2 g (1,125 mol) de carbonate de potassium.

On ajoute 600 g de diméthylformamide et l'ensemble est chauffé sous agitation à une température voisine de 80° C pendant 4 h.

Après retour à une température voisine de 25°C, le mélange réactionnel est filtré sur verre fritté n° 3 et lavé par 75 g de diméthylformamide.

Le filtrat est concentré par évaporation sous pression réduite (25-40 mbars) puis dilué par 150 ml d'eau et extrait, successivement, par 1 fois 150 ml de dichlorométhane et 75 ml de dichlorométhane.

Les phases organiques réunies sont lavées avec 75 ml d'eau et concentrées à sec par évaporation sous pression réduite.

On obtient ainsi 265 g de liquide jaune limpide correspondant à un rendement de 98.9 % en 2-[2-(formyl-phénoxy)]-hexanoate de méthyle titrant 99,6 % de pureté par chromatographie en phase gazeuse.

## Revendications

1. Procédé de préparation des composés nitroaromatiques de formule (I) : 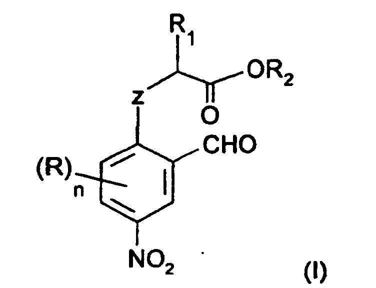 dans laquelle :
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle.
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone,
**caractérisé par le fait qu'**il consiste à effectuer la nitration sélective en position 4 à l'aide d'une source de NO₂⁺ et en présence d'acide sulfurique, d'un composé aromatique répondant à la formule (II) : 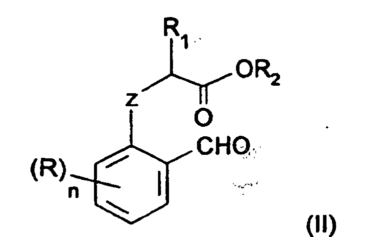 dans ladite formule (II), R, R₁, R₂, Z et n ont la signification donnée précédemment.

2. Procédé selon la revendication 1 **caractérisé par le fait que** le substrat de départ répond à la formule (II) dans laquelle R représente un atome d'hydrogène ou l'un des groupes suivants :
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

3. Procédé selon la revendication 1 **caractérisé par le fait que** le substrat de départ répond à la formule (II) dans laquelle R représente un atome d'hydrogène, un groupe méthyle ou éthyle, un groupe méthoxy ou éthoxy.

4. Procédé selon la revendication 1 **caractérisé par le fait que** le substrat de départ répond à la formule (II) dans laquelle R₁ représente un groupe alkyle ayant de 1 à 4 atomes de carbone.

5. Procédé selon la revendication 1 **caractérisé par le fait que** le substrat de départ répond à la formule (II) dans laquelle R₂ représente un groupe alkyle ayant de 1 à 4 atomes de carbone.

6. Procédé selon la revendication 1 **caractérisé par le fait que** le substrat de départ répond à la formule (II) dans laquelle Z est un atome d'oxygène.

7. Procédé selon la revendication 1 **caractérisé par le fait que** l'on effectue la nitration du composé de formule (II) par réaction de ce dernier avec une source de NO₂⁺, en l'absence ou en présence d'un solvant organique, de préférence, un hydrocarbure halogéné aliphatique et plus préférentiellement le dichlorométhane.

8. Procédé selon la revendication 7 **caractérisé par le fait que** le réactif de nitration est toute source de NO₂⁺, de préférence, du dioxyde d'azote NO₂, de l'anhydride azoteux N₂O₃, du peroxyde d'azote N₂O₄, de l'oxyde d'azote NO associé à un agent oxydant tel que, par exemple, l'acide nitrique, le dioxyde d'azote ou l'oxygène ; l'acide nitreux ; le sulfate de nitrosyle ou un sel nitreux, de préférence un sel de métal alcalin, de préférence, le sodium ; un nitrite d'alkyle.

9. Procédé selon la revendication 7 **caractérisé par le fait que** la quantité de réactif de nitration mise en oeuvre est un mélange sulfonitrique.

10. Procédé selon la revendication 7 **caractérisé par le fait que** la quantité d'acide nitrique exprimée par le rapport molaire composé aromatique O- ou S-alkylé/acide nitrique varie entre 0,9 et 1,1, de préférence, entre 0,95 et 1,05.

11. Procédé selon la revendication 7 **caractérisé par le fait que** la quantité d'acide sulfurique exprimée par le rapport molaire composé aromatique O- ou S-alkylé/acide sulfurique varie entre 0,9 et 1,1, de préférence, entre 0,95 et 1,05.

12. Procédé selon la revendication 7 **caractérisé par le fait que** la température de la réaction de nitration est comprise entre -10°C et 20°C, de préférence entre -5°C et 10°C.

13. Procédé selon l'une des revendications 1 à 12 **caractérisé par le fait que** le composé de formule (II) est obtenu en faisant réagir :
- un composé de type 2-hydroxy- ou 2-thiobenzaldéhyde de formule (III): 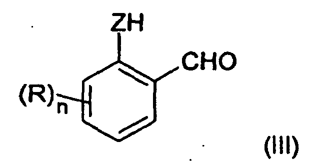 dans ladite formule (III), R, Z et n ont la signification donnée précédemment dans l'une des revendications 1 à 3,
- et un acide carboxylique ou dérivé de formule (IV) : 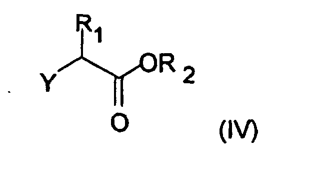 dans ladite formule (IV) :
- Y représente un groupe partant, de préférence, un atome d'halogène ou un groupe ester sulfonique de formule - OSO₂ - 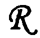 dans lequel 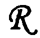 est un groupe hydrocarboné,
- R₁ , R₂ ont la signification donnée précédemment dans l'une des revendications 1, 4 et 5.

14. Procédé selon la revendication 13 **caractérisé par le fait que** le composé de formule (III) est l'aldéhyde salicylique et l'acide carboxylique ou dérivé de formule (IV) est le 2-bromohexanoate de méthyle ou d'éthyle.

15. Procédé selon la revendication 13 **caractérisé par le fait que** le rapport molaire entre le composé de formule (III) et le composé de formule (IV) est choisi entre 1 et 1,2.

16. Procédé selon la revendication 13 **caractérisé par le fait que** l'on fait réagir un composé aromatique de formule (III) avec un acide carboxylique ou dérivé de formule (IV) : la réaction étant conduite en présence d'une base, de préférence dans un solvant organique.

17. Procédé selon la revendication 16 **caractérisé par le fait que** la base peut être une base minérale, de préférence, un sel de métal alcalin ou alcalino-terreux, de préférence, un hydroxyde de métal alcalin ou alcalino-terreux qui peut être l'hydroxyde de sodium, de potassium ou de calcium; un carbonate ou hydrogénocarbonate d'un métal alcalin, de préférence, le carbonate de sodium ou une base organique, de préférence, un hydroxyde d'ammonium quaternaire ou à une amine tertiaire.

18. Procédé selon la revendication 17 **caractérisé par le fait que** la base mise en oeuvre est le carbonate de sodium ou de potassium.

19. Procédé selon la revendication 16 **caractérisé par le fait que** la réaction du composé de formule (III) sous forme salifié et du composé de formule (IV) est conduite dans un solvant organique, choisi préférentiellement parmi les hydrocarbures aromatiques, halogénés ou non, les éther-oxydes aliphatiques, cycloaliphatiques ou aromatiques, les nitriles alphatiques ou aromatiques ; les carboxamides linéaires ou cycliques, de préférence, le N,N-diméthylacétamide ou le diméthylformamide.

20. Procédé selon la revendication 18 **caractérisé par le fait que** l'on ajoute des ions iodure, de préférence, des iodures de métaux alcalins, et préférentiellement l'iodure de potassium.

21. Procédé selon l'une des revendications 13 à 20 **caractérisé par le fait que** la réaction entre le composé de formule (III) et le composé de formule (IV) est conduite à une température comprise entre 0°C et 100°C, de préférence, entre 25°C et 50°C.

22. Procédé selon la revendication 13 **caractérisé par le fait que** l'on fait réagir un composé aromatique de formule (III) et un acide carboxylique ou dérivé, en milieu aqueux, en présence d'une base et d'un catalyseur de transfert de phase.

23. Procédé selon la revendication 22 **caractérisé par le fait que** le catalyseur de transfert de phase est une tris(éther-amine), de préférence la tris(3,3-dioxaheptyl)amine.

24. Procédé selon la revendication 22 **caractérisé par le fait que** le catalyseur de transfert de phase est un sel d'onium dont l'onium répond à l'une des formules suivantes : 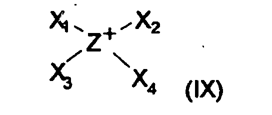 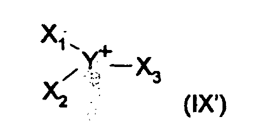 dans lesdites formules:
- Z représente N, P ou As ;
- Y représente S, O, Se ou C ;
- X₁, X₂, X₃ et X₄, identiques ou différents représentent :
. un groupe alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
. un groupe alcényle, linéaire ou ramifié, ayant 2 à 12 atomes de carbone ;
. un groupe aryle ayant 6 à 10 atomes de carbone, éventuellement substitué par un ou plusieurs groupements ou atomes alkyle ayant 1 à 4 atomes de carbone, alkoxy, alkoxycarbonyle, le groupe alkoxy ayant 1 à 4 atomes de carbone, ou halogène,
- deux desdits groupes X₁ à X₄ pouvant former ensemble un groupe alkylène, alcénylène ou alcadiènylène, linéaire ou ramifié ayant de 3 à 6 atomes de carbone.

25. Procédé selon la revendication 24 **caractérisé par le fait que** l'anion desdits sels d'onium est choisi parmi les ions : F⁻, ClO₄⁻, PF₆⁻, BF₄⁻, SnCl₆⁻, SbCl₆⁻, B(Ph)₄⁻, PO₄³⁻, HPO₄²⁻, H₂PO₄⁻, CH₃SO₃⁻, Ph-SO₃⁻, HSO₄⁻, NO₃⁻, SO₄²⁻, Cl⁻, Br⁻, I⁻, OH⁻, Ph représentant un groupe phényle ; l'anion desdits sels d'onium étant choisi de préférence parmi les ions Br⁻, Cl⁻ et OH⁻.

26. Procédé selon la revendication 22 **caractérisé par le fait que** le catalyseur de transfert de phase est choisi parmi : le chlorure ou le bromure de tributylbenzylammonium ou phosphonium, le chlorure ou le bromure de tétraméthylammonium ou phosphonium, le chlorure ou le bromure de tétraéthylammonium ou phosphonium, le chlorure ou le bromure de tétrabutylammonium ou phosphonium et représente de préférence le chlorure ou le bromure de tributylbenzylammonium.

27. Procédé selon la revendication 22 **caractérisé par le fait que** la base est choisie parmi le carbonate de potassium, le carbonate de sodium et l'ammoniaque et est de préférence le carbonate de potassium.

28. Procédé selon la revendication 22 **caractérisé par** le fait le rapport molaire entre ledit catalyseur et le composé de formule (III) varie entre 0,01 et 0,50, de préférence, entre 0,05 et 0,2.

29. Procédé selon la revendication 22 **caractérisé par le fait que** la réaction est conduite en milieu aqueux.

30. Procédé selon la revendication 22 **caractérisé par le fait que** la température réactionnelle varie entre la température ambiante et 80°C, de préférence entre 50°C et 65°C.

31. Procédé de préparation d'un composé hétérocyclique répondant à la formule générale (V) : 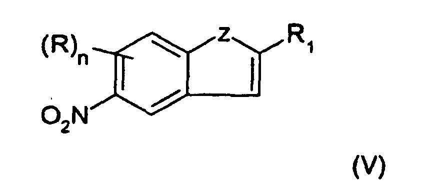 dans la formule (V), R, R₁, Z et n ont la signification donnée précédemment dans l'une des revendications 1 à 4, 6,
**caractérisé par le fait que** l'on effectue une cyclisation du composé de formule (I) décrit dans l'une des revendications 1 à 6 précédée d'une éventuelle saponification du composé de formule (I) quand R₂ est différent de H.

32. Procédé selon la revendication 31 **caractérisé par le fait que** l'on effectue la cyclisation du composé de formule (I) en présence d'acétate de sodium et en milieu anhydride acétique.

33. Procédé selon la revendication 31 **caractérisé par le fait que** l'on effectue la cyclisation du composé de formule (I) en présence de carbonate de sodium ou de potassium et en milieu anhydride acétique.

34. Procédé selon la revendication 31 **caractérisé par le fait qu'**il est obtenu :
- en préparant le composé de formule (II) par réaction du composé de formule (III) et de l'acide carboxylique ou dérivé de formule (IV), en présence d'une base et éventuellement d'un catalyseur de transfert de phase,
- en effectuant la nitration sélective du composé de formule (II) en position 4 selon le procédé décrit dans l'une des revendications 1 à 12,
- en soumettant le composé obtenu de formule (I) à une saponification si nécessaire dans le cas où R₂ est différent de H,
- en effectuant la cyclisation du produit otenu.

35. Procédé selon la revendication 31 **caractérisé par le fait qu'**il est obtenu :
- en préparant le composé de formule (II) par réaction du composé de formule (III) et de l'acide carboxylique ou dérivé de formule (IV), en présence d'une base et éventuellement d'un catalyseur de transfert de phase,
- en soumettant le composé obtenu de formule (II) à une saponification si nécessaire dans le cas où R₂ est différent de H,
- en effectuant la nitration sélective du composé de formule (II) dans laquelle R₂ est un atome d'hydrogène, selon le procédé décrit dans l'une des revendications 1 à 12,
- en conduisant la cyclisation du produit obtenu.

36. Procédé selon l'une des revendications 35 et 36 **caractérisé par le fait que** la cyclisation est conduite selon le procédé décrit dans l'une des revendications 32 et 33.

37. Procédé selon l'une des revendications 31 à 36 **caractérisé par le fait que** le composé de formule (V) est le 2-n-butyl-5-nitrobenzofurane.

38. Nouveaux composés nitroaromatiques répondant à la formule générale : 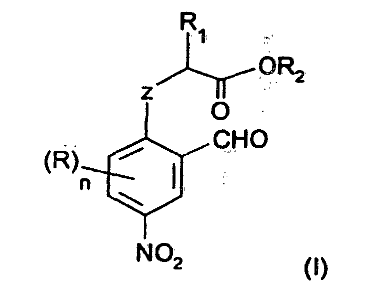 dans laquelle:
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle.
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

39. Nouveaux composés nitroaromatiques selon la revendication 38 **caractérisés par le fait que** R représente un atome d'hydrogène ou l'un des groupes suivants :
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

40. Nouveaux composés nitroaromatiques selon la revendication 38 **caractérisés par le fait que** R représente un atome d'hydrogène, un groupe méthyle ou éthyle, un groupe méthoxy ou éthoxy.

41. Nouveaux composés nitroaromatiques selon la revendication 38 **caractérisés par le fait que** R₁ représente un groupe alkyle ayant de 1 à 4 atomes de carbone.

42. Nouveaux composés nitroaromatiques selon la revendication 38 **caractérisés par le fait que** R₂ représente un groupe alkyle ayant de 1 à 4 atomes de carbone.

43. acide 2-(2-formyl-4-nitro-phénoxy)-hexanoïque 2-(2-formyl-4-nitro-phénoxy)-hexanoate de méthyle ou d'éthyle.

## Patentansprüche

1. Verfahren zur Herstellung nitroaromatischer Verbindungen der Formel (I): 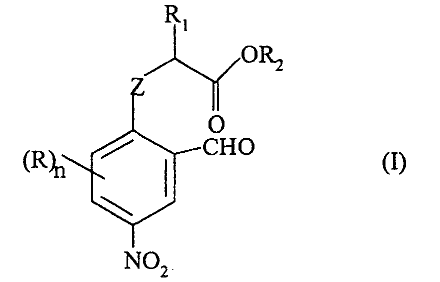 wobei:
- R₁ eine lineare oder verzweigte Alkylgruppe mit 1 bis 12 Kohlenstoffatomen, eine gegebenenfalls mit einer Alkylgruppe mit 1 bis 4 Kohlenstoffatomen substituierte Phenylgruppe oder eine Halogenphenylgruppe darstellt,
- R₂ ein Wasserstoffatom, eine Kohlenwasserstoffgruppe mit 1 bis 12 Kohlenstoffatomen, die eine lineare oder verzweigte Alkylgruppe sein kann, eine Cycloalkylgruppe, eine Phenylgruppe oder eine Phenylalkylgruppe darstellt,
- Z ein Sauerstoff- oder ein Schwefelatom darstellt,
- R ein Wasserstoffatom oder einen Substituenten darstellt,
- n eine ganze Zahl 0, 1, 2 oder 3 ist, vorzugsweise gleich 0 ist,
- wobei, wenn n größer 1 ist, zwei Gruppen R und 2 aufeinanderfolgende Atome des Benzolrings miteinander einen gesättigten, ungesättigten oder aromatischen Ring mit 5 bis 7 Kohlenstoffatomen bilden können,
**dadurch gekennzeichnet, daß** man eine selektive Nitrierung einer aromatischen Verbindung in 4-Position mit Hilfe einer NO₂⁺-Quelle und in Gegenwart von Schwefelsäure durchführt, wobei die aromatische Verbindung der Formel (II) entspricht: 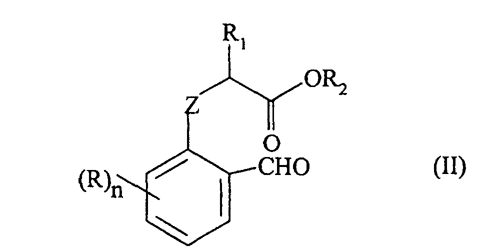 wobei in der Formel (II) R, R₁, R₂, Z und n die zuvor angegebene Bedeutung haben.

2. Verfahren nach Anspruch 1, **dadurch gekennzeichnet, daß** die Ausgangsverbindung der allgemeinen Formel (II) entspricht, wobei R ein Wasserstoffatom oder eine der folgenden Gruppen darstellt:
- eine Hydroxylgruppe,
- eine lineare oder verzweigte Alkylgruppe mit 1 bis 6 Kohlenstoffatomen, vorzugsweise 1 bis 4 Kohlenstoffatomen, wie Methyl, Ethyl, Propyl, Isopropyl, Butyl, Isobutyl, sek.-Butyl und tert.-Butyl,
- eine Alkoxygruppe mit 1 bis 6 Kohlenstoffatomen, vorzugsweise 1 bis 4 Kohlenstoffatomen,
- eine Estergruppe mit 1 bis 10 Kohlenstoffatomen, vorzugsweise 1 bis 4 Kohlenstoffatomen,
- eine Alkylamidgruppe mit 1 bis 6 Kohlenstoffatomen, vorzugsweise 1 bis 4 Kohlenstoffatomen,
- eine Carboxamidgruppe,
- ein Halogenatom,
- eine Trifluormethylgruppe.

3. Verfahren nach Anspruch 1, **dadurch gekennzeichnet, daß** die Ausgangsverbindung der Formel (II) entspricht, wobei R ein Wasserstoffatom, eine Methyl- oder Ethylgruppe oder eine Methoxy- oder Ethoxygruppe darstellt.

4. Verfahren nach Anspruch 1, **dadurch gekennzeichnet, daß** die Ausgangsverbindung der Formel (II) entspricht, wobei R₁ eine Alkylgruppe mit 1 bis 4 Kohlenstoffatomen ist.

5. Verfahren nach Anspruch 1, **dadurch gekennzeichnet, daß** die Ausgangsverbindung der Formel (II) entspricht, wobei R₂ eine Alkylgruppe mit 1 bis 4 Kohlenstoffatomen darstellt.

6. Verfahren nach Anspruch 1, **dadurch gekennzeichnet, daß** die Ausgangsverbindung der Formel (II) entspricht, wobei Z ein Sauerstoffatom ist.

7. Verfahren nach Anspruch 1, **dadurch gekennzeichnet, daß** man die Nitrierung der Verbindung der Formel (II) durch Reaktion der letzteren mit einer NO₂⁺-Quelle in Abwesenheit oder in Gegenwart eines organischen Lösemittels, vorzugsweise eines aliphatischen halogenierten Kohlenwasserstoffs, besonders bevorzugt Dichlormethan, durchführt.

8. Verfahren nach Anspruch 7, **dadurch gekennzeichnet, daß** das Nitrierungsreagenz jede beliebige NO₂⁺-Quelle ist, vorzugsweise Stickstoffdioxid NO₂, Distickstofftrioxid N₂O₃, Distickstofftetroxid N₂O₄ oder Stickstoffmonoxid NO, gemeinsam mit einem Oxidationsmittel, wie z. B. Salpetersäure, Stickstoffdioxid oder Sauerstoff; salpetriger Säure; Nitrosylsulfat oder einem salpetrigem Salz, vorzugsweise einem Alkalimetallsalz, vorzugsweise Natrium; einem Alkylnitrit.

9. Verfahren nach Anspruch 7, **dadurch gekennzeichnet, daß** die eingesetzte Menge an Nitrierungsreagenz eine Sulfonitrierungsmischung (Schwefelsäure/Salpetersäure-Mischung) ist.

10. Verfahren nach Anspruch 7, **dadurch gekennzeichnet, daß** die Salpetersäuremenge, berechnet als Molverhältnis der O- oder S-alkylierten aromatischen Verbindung/Salpetersäure, zwischen 0,9 und 1,1, vorzugsweise zwischen 0,95 und 1,05, variiert.

11. Verfahren nach Anspruch 7, **dadurch gekennzeichnet, daß** die Schwefelsäuremenge, berechnet als Molverhältnis der O- oder S-alkylierten aromatischen Verbindung/Schwefelsäure, zwischen 0,9 und 1,1, vorzugsweise zwischen 0,95 und 1,05, variiert.

12. Verfahren nach Anspruch 7, **dadurch gekennzeichnet, daß** die Temperatur für die Nitrierungsreaktion zwischen -10 °C und 20 °C, vorzugsweise zwischen -5 °C und 10 °C, liegt.

13. Verfahren nach einem der Ansprüche 1 bis 12, **dadurch gekennzeichnet, daß** die Verbindung der Formel (II) erhalten wird durch Reaktion
- einer Verbindung vom Typ 2-Hydroxy- oder 2-Thiobenzaldehyd der Formel (III): 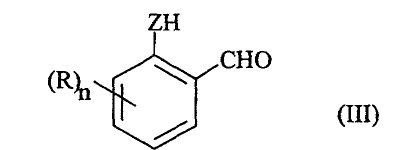 wobei in der Formel (III) R, Z und n die zuvor in einem der Ansprüche 1 bis 3 angegebene Bedeutung haben,
- und einer Carbonsäure oder ihres Derivats der Formel (IV): 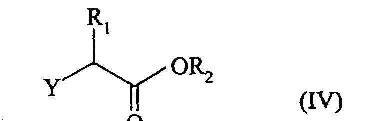 wobei in der Formel (IV):
- Y eine Abgangsgruppe darstellt, vorzugsweise ein Halogenatom oder eine Sulfonestergruppe der Formel -OSO₂- 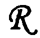 , wobei 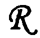 eine Kohlenwasserstoffgruppe ist,
- R₁ und R₂ die zuvor in einem der Ansprüche 1, 4 und 5 angegebene Bedeutung haben.

14. Verfahren nach Anspruch 13, **dadurch gekennzeichnet, daß** die Verbindung der Formel (III) Salicylaldehyd ist und die Carbonsäure der Formel (IV) oder ihr Derivat 2-Bromhexansäuremethyl- oder -ethylester ist.

15. Verfahren nach Anspruch 13, **dadurch gekennzeichnet, daß** das Molverhältnis zwischen der Verbindung der Formel (III) und der Verbindung der Formel (IV) zwischen 1 und 1,2 ausgewählt ist.

16. Verfahren nach Anspruch 13, **dadurch gekennzeichnet, daß** man eine aromatische Verbindung der Formel (III) mit einer Carbonsäure der Formel (IV) oder ihrem Derivat reagieren läßt, wobei die Reaktion in Gegenwart einer Base, vorzugsweise in einem organischen Lösemittel, durchgeführt wird.

17. Verfahren nach Anspruch 16, **dadurch gekennzeichnet, daß** die Base eine anorganische Base sein kann, vorzugsweise ein Alkali- oder Erdalkalisalz, vorzugsweise ein Alkalimetall- und Erdalkalimetallhydroxid, welches Natrium-, Kalium- oder Calciumhydroxid sein kann; ein Alkalimetallcarbonat oder -hydrogencarbonat, vorzugsweise Natriumcarbonat; oder eine organische Base sein kann, vorzugsweise ein quarternäres Ammoniumhydroxid oder ein tertiäres Amin.

18. Verfahren nach Anspruch 17, **dadurch gekennzeichnet, daß** die eingesetzte Base Natrium- oder Kaliumcarbonat ist.

19. Verfahren nach Anspruch 16, **dadurch gekennzeichnet, daß** die Reaktion der Verbindung der Formel (III) in versalzter Form und der Verbindung der Formel (IV) in einem organischen Lösemittel durchgeführt ist, welches vorzugsweise ausgewählt ist aus gegebenenfalls halogenierten aromatischen Kohlenwasserstoffen, aliphatischen, cycloaliphatischen oder aromatischen Etheroxiden, aliphatischen oder aromatischen Nitrilen; linearen oder ringförmigen Carboxamiden, vorzugsweise N,N-Dimethylacetamid oder Dimethylformamid.

20. Verfahren nach Anspruch 18, **dadurch gekennzeichnet, daß** man Iodidionen hinzugibt, vorzugsweise Alkalimetalliodide und bevorzugt Kaliumiodid.

21. Verfahren nach einem der Ansprüche 13 bis 20, **dadurch gekennzeichnet, daß** die Reaktion zwischen der Verbindung der Formel (III) und der Verbindung der Formel (IV) bei einer Temperatur zwischen 0 °C und 100 °C, vorzugsweise zwischen 25 °C und 50 °C, durchgeführt wird.

22. Verfahren nach Anspruch 13, **dadurch gekennzeichnet, daß** man eine aromatische Verbindung der Formel (III) und eine Carbonsäure oder ihr Derivat in wäßrigem Milieu in Gegenwart einer Base und eines Phasentransferkatalysators reagieren läßt.

23. Verfahren nach Anspruch 22, **dadurch gekennzeichnet, daß** der Phasentransferkatalysator ein Tris(etheramin), vorzugsweise Tris(3,3-dioxaheptyl)amin, ist.

24. Verfahren nach Anspruch 22, **dadurch gekennzeichnet, daß** der Phasentransferkatalysator ein Oniumsalz ist, wobei das Onium einer der folgenden Formeln entspricht: 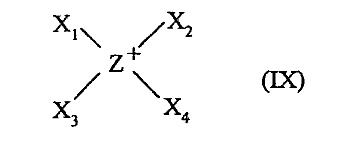 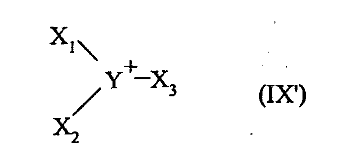 wobei in diesen Formeln
- Z N, P oder As darstellt;
- Y S, O, Se oder C darstellt;
- X₁, X₂, X₃ und X₄, identisch oder verschieden, darstellen:
• eine lineare oder verzweigte Alkylgruppe mit 1 bis 16 Kohlenstoffatomen, die gegebenenfalls substituiert ist mit einer bzw. einem oder mehreren der folgenden Gruppen oder Atome: Phenyl, Hydroxyl, Halogen, Nitro, Alkoxy oder Alkoxycarbonyl, wobei die Alkoxygruppen 1 bis 4 Kohlenstoffatome aufweisen;
• eine lineare oder verzweigte Alkenylgruppe mit 2 bis 12 Kohlenstoffatomen;
• eine Arylgruppe mit 6 bis 10 Kohlenstoffatomen, die gegebenenfalls substituiert ist mit einer bzw. einem oder mehreren der folgenden Gruppen oder Atome: Alkyl mit 1 bis 4 Kohlenstoffatomen, Alkoxy oder Alkoxycarbonyl, wobei die Alkoxygruppe 1 bis 4 Kohlenstoffatome aufweist, oder Halogen;
- wobei die Gruppen X₁ bis X₄ gemeinsam eine lineare oder verzweigte Alkylen-, Alkenylen- oder Alkadienylengruppe mit 3 bis 6 Kohlenstoffatomen bilden können.

25. Verfahren nach Anspruch 24, **dadurch gekennzeichnet, daß** das Anion dieser Oniumsalze ausgewählt ist aus den folgenden Ionen: F⁻, ClO₄⁻, PF₆⁻, BF₄⁻, SnCl₆⁻, SbCl₆⁻, B(Ph)₄⁻, PO₄³⁻, HPO₄²⁻, H₂PO₄⁻, CH₃SO₃⁻, Ph-SO₃⁻, HSO₄⁻, NO₃⁻, SO₄²⁻, Cl⁻, Br⁻, I⁻ und OH⁻, wobei Ph eine Phenylgruppe darstellt, wobei das Anion dieser Oniumsalze vorzugsweise ausgewählt ist aus den Ionen Br⁻, Cl⁻ und OH⁻.

26. Verfahren nach Anspruch 22, **dadurch gekennzeichnet, daß** der Phasentransferkatalysator ausgewählt ist aus Tributylbenzylammonium oder -phosphoniumchlorid oder -bromid, Tetramethylammonium oder -phosphoniumchlorid oder -bromid, Tetraethylammonium oder -phosphoniumchlorid oder -bromid, Tetrabutylammonium oder -phosphoniumchlorid oder -bromid und vorzugsweise Tributylbenzylammoniumchlorid oder -bromid darstellt.

27. Verfahren nach Anspruch 22, **dadurch gekennzeichnet, daß** die Base ausgewählt ist aus Kaliumcarbonat, Natriumcarbonat und Ammoniak und vorzugsweise Kaliumcarbonat ist.

28. Verfahren nach Anspruch 22, **dadurch gekennzeichnet, daß** das Molverhältnis zwischen Katalysator und der Verbindung der Formel (III) zwischen 0,01 und 0,50, vorzugsweise zwischen 0,05 und 0,2, variiert.

29. Verfahren nach Anspruch 22, **dadurch gekennzeichnet, daß** die Reaktion in wäßrigem Milieu durchgeführt wird.

30. Verfahren nach Anspruch 22, **dadurch gekennzeichnet, daß** die Reaktionstemperatur zwischen Umgebungstemperatur und 80 °C, vorzugsweise zwischen 50 °C und 65 °C, variiert.

31. Verfahren zur Herstellung einer heterocyclischen Verbindung der allgemeinen Formel (V) 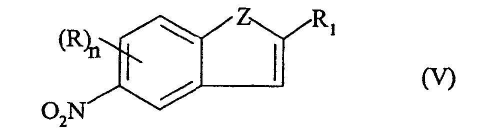 wobei in der Formel (V) R, R₁, Z und n die zuvor in einem der Ansprüche 1 bis 4 und 6 angegebene Bedeutung haben,
**dadurch gekennzeichnet, daß** man eine Cyclisierung (Ringbildung) der Verbindung der Formel (I), wie in einem der Ansprüche 1 bis 6 beschrieben, durchführt, der eine gegebenenfalls durchgeführte Verseifung der Verbindung der Formel (I) vorangeht, wenn R₂ von H verschieden ist.

32. Verfahren nach Anspruch 31, **dadurch gekennzeichnet, daß** man die Cyclisierung der Verbindung der Formel (I) in Gegenwart von Natriumacetat und in Essigsäureanhydridmilieu durchführt.

33. Verfahren nach Anspruch 31, **dadurch gekennzeichnet, daß** man die Cyclisierung der Verbindung der Formel (I) in Gegenwart von Natrium- oder Kaliumcarbonat und in Essigsäureanydridmilieu durchführt.

34. Verfahren nach Anspruch 31, **dadurch gekennzeichnet, daß** sie erhalten wird,
- indem man eine Verbindung der Formel (II) durch Reaktion der Verbindung der Formel (III) und der Carbonsäure der Formel (IV) oder ihres Derivats in Gegenwart einer Base und gegebenenfalls eines Phasentransferkatalysators durchführt,
- indem man eine selektive Nitrierung der Verbindung der Formel (II) in 4-Position nach dem in einem der Ansprüche 1 bis 12 beschriebenen Verfahren durchführt,
- indem man die erhaltene Verbindung der Formel (I) erforderlichenfalls einer Verseifung unterwirft für den Fall, daß R₂ von H verschieden ist,
- indem man die Cyclisierung des erhaltenen Produkts durchführt.

35. Verfahren nach Anspruch 31, **dadurch gekennzeichnet, daß** sie erhalten wird,
- indem man die Verbindung der Formel (II) durch Reaktion der Verbindung der Formel (III) und der Carbonsäure der Formel (IV) oder ihres Derivats in Gegenwart einer Base und gegebenenfalls eines Phasentransferkatalysators herstellt,
- indem man die erhaltene Verbindung der Formel (II) erforderlichenfalls einer Verseifung unterwirft für den Fall, daß R₂ von H verschieden ist,
- indem man eine selektive Nitrierung der Verbindung der Formel (II), wobei in der Formel (II) R₂ ein Wasserstoffatom ist, gemäß dem in einem der Ansprüche 1 bis 12 beschriebenen Verfahren durchführt,
- indem man die Cyclisierung des erhaltenen Produkts durchführt.

36. Verfahren nach Anspruch 34 oder 35, **dadurch gekennzeichnet, daß** die Cyclisierung gemäß dem in einem der Ansprüche 32 und 33 beschriebenen Verfahren durchgeführt wird.

37. Verfahren nach einem der Ansprüche 31 bis 36, **dadurch gekennzeichnet, daß** die Verbindung der Formel (V) 2-n-Butyl-5-nitrobenzofuran ist.

38. Neue nitroaromatische Verbindungen der allgemeinen Formel 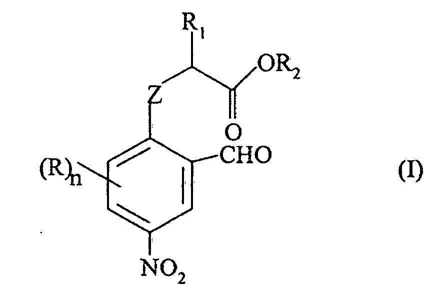 wobei:
- R₁ eine lineare oder verzweigte Alkylgruppe mit 1 bis 12 Kohlenstoffatomen, eine gegebenenfalls mit einer Alkylgruppe mit 1 bis 4 Kohlenstoffatomen substituierte Phenylgruppe oder eine Halogenphenylgruppe darstellt,
- R₂ ein Wasserstoffatom, eine Kohlenwasserstoffgruppe mit 1 bis 12 Kohlenstoffatomen, die eine lineare oder verzweigte Alkylgruppe sein kann, eine Cycloalkylgruppe, eine Phenylgruppe oder eine Phenylalkylgruppe darstellt,
- Z ein Sauerstoff- oder ein Schwefelatom darstellt,
- R ein Wasserstoffatom oder einen Substituenten darstellt,
- n eine ganze Zahl 0, 1, 2 oder 3 ist, vorzugsweise gleich 0 ist,
- wobei, wenn n größer 1 ist, zwei Gruppen R und 2 aufeinanderfolgende Atome des Benzolrings miteinander einen gesättigten, ungesättigten oder aromatischen Ring mit 5 bis 7 Kohlenstoffatomen bilden können,

39. Neue nitroaromatische Verbindungen nach Anspruch 38, **dadurch gekennzeichnet, daß** R ein Wasserstoffatom oder eine der folgenden Gruppen darstellt:
- eine Hydroxylgruppe,
- eine lineare oder verzweigte Alkylgruppe mit 1 bis 6 Kohlenstoffatomen, vorzugsweise 1 bis 4 Kohlenstoffatomen, wie Methyl, Ethyl, Propyl, Isopropyl, Butyl, Isobutyl, sek.-Butyl und tert.-Butyl,
- eine Alkoxygruppe mit 1 bis 6 Kohlenstoffatomen, vorzugsweise 1 bis 4 Kohlenstoffatomen,
- eine Estergruppe mit 1 bis 10 Kohlenstoffatomen, vorzugsweise 1 bis 4 Kohlenstoffatomen,
- eine Alkylamidgruppe mit 1 bis 6 Kohlenstoffatomen, vorzugsweise 1 bis 4 Kohlenstoffatomen,
- eine Carboxamidgruppe,
- ein Halogenatom,
- eine Trifluormethylgruppe.

40. Neue nitroaromatische Verbindungen nach Anspruch 38, **dadurch gekennzeichnet, daß** R ein Wasserstoffatom, eine Methyl- oder Ethylgruppe oder eine Methoxy- oder Ethoxygruppe darstellt.

41. Neue nitroaromatische Verbindungen nach Anspruch 38, **dadurch gekennzeichnet, daß** R₁ eine Alkylgruppe mit 1 bis 4 Kohlenstoffatomen darstellt.

42. Neue nitroaromatische Verbindungen nach Anspruch 38, **dadurch gekennzeichnet, daß** R₂ eine Alkylgruppe mit 1 bis 4 Kohlenstoffatomen darstellt.

43. 2-(2-Formyl-4-nitrophenoxy)hexansäure oder 2-(2-Formyl-4-nitrophenoxy)hexansäuremethyl- oder -ethylester.

## Claims

1. A process for preparing nitroaromatic compounds with formula (I): 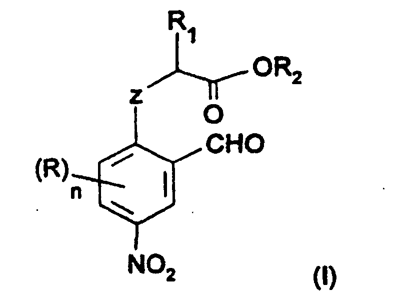 in which:
• R₁ represents a linear or branched alkyl group containing 1 to 12 carbon atoms, a phenyl group that may be substituted by an alkyl group containing 1 to 4 carbon atoms, or a halogenophenyl group;
• R₂ represents a hydrogen atom, a hydrocarbon group containing 1 to 12 carbon atoms, which may be a linear or branched alkyl group, a cycloalkyl group, a phenyl group or a phenylalkyl group;
• Z represents an oxygen or sulphur atom;
• R represents a hydrogen atom or a substituent;
• n is a number equal to 0. 1, 2 or 3, preferably 0;
• when n is greater than 1, two groups R and the successive 2 atoms of the benzene ring can together form a saturated, unsaturated or aromatic cycle containing 5 to 7 carbon atoms;
**characterized in that** it consists of carrying out selective nitration in the 4 position, using a source of NO₂⁺ and in the presence of sulphuric acid, of an aromatic compound with formula (II): 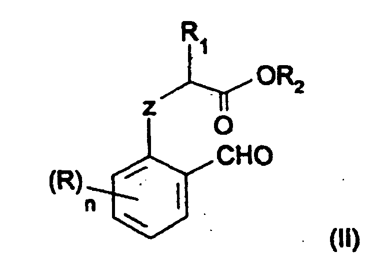 in which formula (II), R, R₁, R₂, Z and n have the meanings given above.

2. A process according to claim 1, **characterized in that** the starting substrate has formula (II), in which R represents a hydrogen atom or one of the following groups:
• a hydroxyl group;
• a linear or branched alkyl group containing 1 to 6 carbon atoms, preferably 1 to 4 carbon atoms, such as methyl, ethyl, propyl, isopropyl, butyl, isobutyl, sec-butyl, tert-butyl;
• an alkoxy group containing 1 to 6 carbon atoms, preferably 1 to 4 carbon atoms;
• an ester group containing 1 to 10 carbon atoms, preferably 1 to 4 carbon atoms;
• an alkylamide group containing 1 to 6 carbon atoms, preferably 1 to 4 carbon atoms;
• a carboxamide group;
• a halogen atom;
• a trifluoromethyl group.

3. A process according to claim 1, **characterized in that** the starting substrate has formula (II) in which R represents a hydrogen atom, a methyl or ethyl group, or a methoxy or ethoxy group.

4. A process according to claim 1, **characterized in that** the starting substrate has formula (II) in which R₁ represents an alkyl group containing 1 to 4 carbon atoms.

5. A process according to claim 1, **characterized in that** the starting substrate has formula (II) in which R₂ represents an alkyl group containing 1 to 4 carbon atoms.

6. A process according to claim 1, **characterized in that** the starting substrate has formula (II) in which Z is an oxygen atom.

7. A process according to claim 1, **characterized in that** nitration of the compound with formula (II) is carried out by reacting it with a source of NO₂⁺ in the presence or absence of an organic solvent, preferably an aliphatic halogenated hydrocarbon, more preferably dichloromethane.

8. A process according to claim 7, **characterized in that** the nitrating reagent is any source of NO₂⁺ , preferably nitrogen dioxide NO₂, nitrous anhydride N₂O₃, dinitrogen tetroxide N₂O₄, nitric oxide NO associated with an oxidising agent such as nitric acid, nitrogen dioxide or oxygen; nitrous acid, nitrosyl sulphate or a nitrous salt, preferably an alkali metal salt, still more preferably, sodium or an alkyl nitrite.

9. A process according to claim 7, **characterized in that** the quantity of nitrating reagent employed is a nitrating mixture.

10. A process according to claim 7, **characterized in that** the quantity of nitric acid, expressed as the mole ratio of the aromatic O- or S-alkylated compound/nitric acid is in the range 0.9 to 1.1, preferably in the range 0.95 to 1.05.

11. A process according to claim 7, **characterized in that** the quantity of sulphuric acid, expressed as the mole ratio of the aromatic O- or S-alkylated compound/sulphuric acid is in the range 0.9 to 1.1, preferably in the range 0.95 to 1.05.

12. A process according to claim 7, **characterized in that** the nitration reaction temperature is in the range -10°C to 20°C, preferably in the range -5°C to 10°C.

13. A process according to one of claims 1 to 12, **characterized in that** the compound with formula (II) is obtained by reacting:
• a compound of the 2-hydroxy- or 2-thiobenzaldehyde type with formula (III): 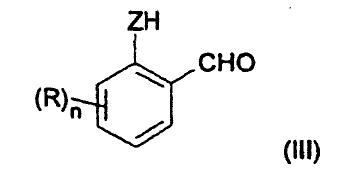 in which formula (III), R, Z and n have the meanings given above in any one of claims 1 to 3;
• and a carboxylic acid or a derivative with formula (IV): 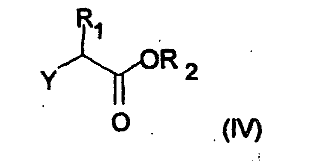 in which formula (IV):
• Y represents a leaving group, preferably a halogen atom or a sulphonic ester group with formula -OSO₂- 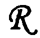 where 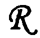 is a hydrocarbon group;
• R₁, R₂ have the meanings given above in any one of claims 1, 4 and 5.

14. A process according to claim 13, **characterized in that** the compound with formula (III) is salicylic anhydride and the carboxylic acid or derivative with formula (IV) is methyl or ethyl 2-bromohexanoate.

15. A process according to claim 13, **characterized in that** mole ratio between the compound with formula (III) and the compound with formula (IV) is between 1 and 1.2.

16. A process according to claim 13, **characterized in that** an aromatic compound with formula (III) is reacted with a carboxylic acid or a derivative with formula (IV), the reaction being carried out in the presence of a base, preferably in an organic solvent.

17. A process according to claim 16, **characterized in that** the base can be a mineral base, preferably an alkali metal or alkaline-earth metal salt, preferably an alkali or alkaline-earth metal hydroxide which may be sodium, potassium or calcium hydroxide; or an alkali metal carbonate or bicarbonate, preferably sodium carbonate; or an organic base, preferably a quaternary ammonium hydroxide or a tertiary amine.

18. A process according to claim 17, **characterized in that** the base employed is sodium or potassium carbonate.

19. A process according to claim 16, **characterized in that** the compound with formula (III) in its salt form and the compound with formula (IV) are reacted in an organic solvent preferably selected from aromatic hydrocarbons that may or may not be halogenated, aliphatic, cycloaliphatic or aromatic ether-oxides, aliphatic or aromatic nitriles; linear or cyclic carboxamides, preferably N,N-dimethylacetamide or dimethylformamide.

20. A process according to claim 18, **characterized in that** iodide ions are added, preferably alkali metal iodides, more preferably potassium iodide.

21. A process according to any one of claims 13 to 20, **characterized in that** the reaction between the compound with formula (III) and the compound with formula (IV) is carried out at a temperature in the range 0°C to 100°C, preferably in the range 25°C to 50°C.

22. A process according to claim 13, **characterized in that** an aromatic compound with formula (III) is reacted with a carboxylic acid or a derivative thereof, in an aqueous medium, in the presence of a base and a phase transfer catalyst.

23. A process according to claim 22, **characterized in that** the phase transfer catalyst is a tris(ether-amine), preferably tris(3,3-dioxaheptyl)amine.

24. A process according to claim 22, **characterized in that** the phase transfer catalyst is an onium salt wherein the onium has one of the following formulae: 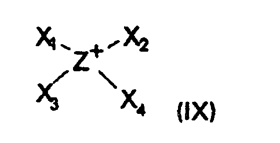 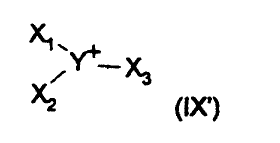 in which formulae:
• Z represents N, P or As;
• Y represents S, O, Se or C;
• X₁, X₂, X₃ and X₄, which may be identical or different, represent:
• a linear or branched alkyl group containing 1 to 16 carbon atoms, optionally substituted by one or more phenyl, hydroxyl, halogen, nitro, alkoxy or alkoxycarbonyl groups, the alkoxy groups containing 1 to 4 carbon atoms;
• a linear or branched alkenyl group containing 2 to 12 carbon atoms;
• an aryl group containing 6 to 10 carbon atoms, optionally substituted by one or more alkyl groups containing 1 to 4 carbon atoms, an alkoxy group, an alkoxycarbonyl group, the alkoxy group containing 1 to 4 carbon atoms, or a halogen;
• two of said groups X₁ to X₄ can together form a linear or branched alkylene, alkenylene or alkadienylene group containing 3 to 6 carbon atoms.

25. A process according to claim 24, **characterized in that** the anion of said onium salts is selected from the following ions: F⁻, ClO₄⁻, PF₆⁻, BF₄⁻, SnCl₆⁻, SbCl₆⁻, B(Ph)₄⁻, PO₄³⁻, HPO₄²⁻, H₂PO₄⁻, CH₃SO₃⁻, Ph-SO₃⁻, HSO₄⁻, NO₃⁻, SO₄²⁻, Cl⁻, Br⁻, I⁻, OH⁻, Ph representing a phenyl group; the anion for said onium salts preferably being selected from Br⁻, Cl⁻ and OH⁻ ions.

26. A process according to claim 22, **characterized in that** the phase transfer catalyst is selected from: tributylbenzylammonium or phosphonium chloride or bromide; tetramethylammonium or phosphonium chloride or bromide; tetraethylammonium or phosphonium chloride or bromide, tetrabutylammonium or phosphonium chloride or bromide, and preferably represents tributylbenzylammonium chloride or bromide.

27. A process according to claim 22, **characterized in that** the base is selected from potassium carbonate, sodium carbonate and ammonia, preferably potassium carbonate.

28. A process according to claim 22, **characterized in that** the mole ratio between said catalyst and the compound with formula (III) is in the range 0.01 to 0.50, preferably in the range 0.05 to 0.2.

29. A process according to claim 22, **characterized in that** the reaction is carried out in an aqueous medium.

30. A process according to claim 22, **characterized in that** the reaction temperature is between ambient temperature and 80°C, preferably between 50°C and 65°C.

31. A process for preparing a heterocyclic compound with general formula (V): 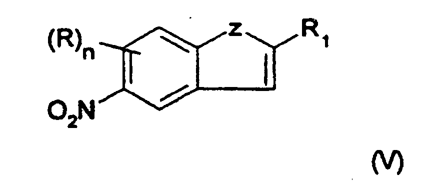 in which formula (V), R, R₁, Z and n have the meanings given above in any one of claims 1 to 4, 6;
**characterized in that** the compound with formula (I) defined in one of the claims 1 to 6 is cyclised, preceded by optional saponification of the compound with formula (I) when R₂ is other than H.

32. A process according to claim 31, **characterized in that** the compound with formula (I) is cyclised in the presence of sodium acetate and in an acetic anhydride medium.

33. A process according to claim 31, **characterized in that** the compound with formula (I) is cyclised in the presence of sodium or potassium carbonate and in an acetic anhydride medium.

34. A process according to claim 31, **characterized in that** it is obtained:
• by preparing the compound with formula (II) by reacting a compound with formula (III) with a carboxylic acid or derivative thereof with formula (IV) in the presence of a base and optionally, a phase transfer catalyst;
• by carrying out selective nitration of the compound with formula (II) in the 4 position using the process described in any one of claims 1 to 12;
• if necessary, by saponifying the compound obtained with formula (I) in the case when R₂ is other than H;
• cyclising the product obtained.

35. A process according to claim 31, **characterized in that** it is obtained:
• by preparing the compound with formula (II) by reacting a compound with formula (III) with a carboxylic acid or derivative thereof with formula (IV) in the presence of a base and optionally, a phase transfer catalyst;
• if necessary, by saponifying the compound obtained with formula (II) in the case when R₂ is other than H;
• by carrying out selective nitration of the compound with formula (II) in which R₂ is a hydrogen atom, using the process described in any one of claims 1 to 12;
• cyclising the product obtained.

36. A process according to claim 35 or claim 36, **characterized in that** cyclisation is carried out using the process defined in claim 32 or claim 33.

37. A process according to any one of claims 31 to 26, **characterized in that** the compound with formula (V) is 2-n-butyl-5-nitrobenzofuran.

38. Novel nitroaromatic compounds with general formula: 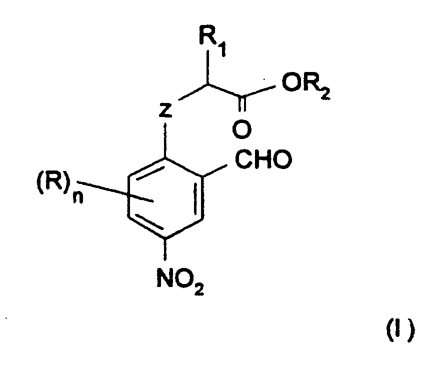 in which:
• R ₁ represents a linear or branched alkyl group containing 1 to 12 carbon atoms, a phenyl group that may be substituted by an alkyl group containing 1 to 4 carbon atoms, or a halogenophenyl group;
• R₂ represents a hydrogen atom, a hydrocarbon group containing 1 to 12 carbon atoms, which may be a linear or branched alkyl group, a cycloalkyl group, a phenyl group or a phenylalkyl group;
• Z represents an oxygen or sulphur atom;
• R represents a hydrogen atom or a substituent;
• n is a number equal to 0. 1, 2 or 3, preferably 0;
• when n is greater than 1, two groups R and the successive 2 atoms of the benzene ring can together form a saturated, unsaturated or aromatic cycle containing 5 to 7 carbon atoms;

39. Novel nitroaromatic compounds according to claim 38, **characterized in that** R represents a hydrogen atom or one of the following groups:
• a hydroxyl group;
• a linear or branched alkyl group containing 1 to 6 carbon atoms, preferably 1 to 4 carbon atoms, such as methyl, ethyl, propyl, isopropyl, butyl, isobutyl, sec-butyl, tert-butyl;
• an alkoxy group containing 1 to 6 carbon atoms, preferably I to 4 carbon atoms;
• an ester group containing 1 to 10 carbon atoms, preferably 1 to 4 carbon atoms;
• an alkylamide group containing 1 to 6 carbon atoms, preferably 1 to 4 carbon atoms;
• a carboxamide group;
• a halogen atom;
• a trifluoromethyl group.

40. Novel nitroaromatic compounds according to claim 38, **characterized in that** R represents a hydrogen atom, a methyl or ethyl group, or a methoxy or ethoxy group.

41. Novel nitroaromatic compounds according to claim 38, **characterized in that** R₁ represents an alkyl group containing 1 to 4 carbon atoms.

42. Novel nitroaromatic compounds according to claim 38, **characterized in that** R₂ represents an alkyl group containing 1 to 4 carbon atoms.

43. 2-(2-formyl-4-nitro-phenoxy)-hexanoic acid;
methyl or ethyl 2-(2-formyl-4-nitro-phenoxy)-hexanoate.
